(19) Europäisches Patentamt
European Patent Office
Office européen des brevets

(11) **EP 3 304 037 B1**

(12) **EUROPEAN PATENT SPECIFICATION**

(45) Date of publication and mention
of the grant of the patent:
**18.12.2019 Bulletin 2019/51**

(51) Int Cl.:
**G01N 15/10** $^{(2006.01)}$    **C12M 1/00** $^{(2006.01)}$

(21) Application number: **16728867.9**

(22) Date of filing: **30.05.2016**

(86) International application number:
**PCT/EP2016/062129**

(87) International publication number:
**WO 2016/193200 (08.12.2016 Gazette 2016/49)**

(54) **METHOD FOR PICKING A COLONY OF CELLS**

VERFAHREN ZUM PICKING VON ZELLKOLONIEN

PROCÉDÉ POUR REPIQUER UNE COLONIE DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.06.2015 US 201562169735 P**

(43) Date of publication of application:
**11.04.2018 Bulletin 2018/15**

(73) Proprietors:
• **Baxalta Incorporated**
  **Bannockburn, IL 60015 (US)**
• **Baxalta GmbH**
  **6300 Zug (CH)**

(72) Inventors:
• **KOEHN, Jadranka**
  **88471 Laupheim (DE)**
• **NEURATH, Marianne**
  **2201 Gerasdorf / Vienna (AT)**

• **BOEHM, Ernst**
  **1210 Vienna (AT)**
• **DOCKAL, Michael**
  **1150 Vienna (AT)**

(74) Representative: **Hoffmann Eitle**
  **Patent- und Rechtsanwälte PartmbB**
  **Arabellastraße 30**
  **81925 München (DE)**

(56) References cited:
• **JEFF JIA CHENG HOU ET AL: "High-throughput ClonePix FL analysis of mAb-expressing clones using the UCOE expression system", NEW BIOTECHNOLOGY, vol. 31, no. 3, 8 February 2014 (2014-02-08), pages 214-220, XP055295684, NL ISSN: 1871-6784, DOI: 10.1016/j.nbt.2014.02.002**
• **NOBUO YOSHIMOTO ET AL: "An automated system for high-throughput single cell-based breeding", SCIENTIFIC REPORTS, vol. 3, 1 February 2013 (2013-02-01), XP055295687, DOI: 10.1038/srep01191**

## Description

### FIELD OF THE INVENTION

The invention relates to a method for picking a colony of cells and producing a cell line from this colony. The disclosure further provides the cell line thus established, and use of the cell line in a method for producing a biological molecule of interest.

### BACKGROUND

Single-cell derived production cell lines are desirable for the production of recombinant proteins, as such cell lines can contribute to product homogeneity and consistency over the whole cultivation period within a production run. The technical and regulatory need for single cell-derived cell lines is exemplified in the following documents:

The ICH guideline Q5D Quality of Biotechnological Products: Derivation and Characterisation of Cell Substrates Used for Production of Biotechnological/Biological Products state that, for recombinant products, the cell substrate is the transfected cell containing the desired sequences, which has been cloned from a single cell progenitor.

In the FDA Supplement to the Points to Consider in the Production and Testing of New Drugs and Biologic& Produced by Recombinant DNA Technology: Nucleic Acid Characterization and Genetic Stability, it is stated that a single host cell containing the expression construct is cloned to give rise to the master cell bank (MCB).

The ICH guideline Q5B Quality of Biotechnological Products: Analysis of the Expression Construct in Cells Used for Production of r-DNA Derived Protein Products convey that the MCB is generally derived from the selected cell clone containing the expression construct.

In Lai et al., 2013, Pharmaceuticals 6: 579-603, Advances in Mammalian Cell Line Development Technologies for Recombinant Protein Production, it is pointed out that, in addition to being a regulatory requirement, single cell cloning or limiting dilution is technically necessary in the process because the protein productivity of individual cells in the transfected and gene amplified cell populations are varied.

There are several methods and automated systems available to achieve this goal in cell line development, including limited dilution cloning, cloning by fluorescence activated cell sorting (FACS), and colony picking from semi-solid medium (e.g. Aviso / ALS, Clonepix / Molecular Devices, ClonaCell Easypick / Stem Cell/Hamilton).

Yoshimoto et al., 2013, Scientific Reports 3:1191, describe an automated system for high-throughput single cell-based breeding. Hou et al., 2014, New Biotechnology 31(3):214-220, describe a high-throughput ClonePix FL analysis of monoclonal antibody (mAb) expressing clones.

To the extent that colony picking from semi-solid medium is concerned, which the invention relates to, the above-mentioned and other known methods and technologies share a major limitation: Using known methods and technologies, it has not been possible to determine whether a given colony (to be) picked from semi-solid medium is derived from a single cell or from more than one cell. In other words, it has not been possible to verify whether a given colony is monoclonal when picking the colony from semi-solid medium following known protocols.

In view of the regulatory and technical requirement to use single-cell derived cell lines in many industrial processes, such as the production of recombinant proteins, and in view of the limitations of known technologies for development of such cell lines, there is a constant need for improved technologies to develop single cell-derived cell lines.

### SUMMARY OF THE INVENTION

The present invention relates to the following items:

1. A method for picking a colony of cells, the method comprising

(a) providing a suspension comprising cells and semi-solid medium in a sample container, the sample container having a substantially flat bottom wall,
(b) centrifuging the sample container such that, after centrifugation, substantially all cells are positioned at the bottom wall,
(c) determining and recording the position of one or more cells located within at least one predetermined region at the bottom wall,
(d) incubating the sample container under conditions suitable for the growth of colonies of cells,
(e) determining and recording the area occupied by one or more colonies of cells, the one or more colonies of cells being located within the at least one predetermined region at the bottom wall, and
(f) selecting and picking at least one of the one or more colonies of cells occupying the area or areas determined and recorded in (e).

2. A method for determining the clonal status of a picked colony of cells, the method comprising

(a) providing a suspension comprising cells and semi-solid medium in a sample container, the sample container having a substantially flat bottom wall,
(b) centrifuging the sample container such that, after centrifugation, substantially all cells are positioned at the bottom wall,
(c) determining and recording the position of one or more cells located within at least one predetermined region at the bottom wall,
(d) incubating the sample container under conditions suitable for the growth of colonies of cells,
(e) determining and recording the area occupied by one or more colonies of cells, the one or more colonies of cells being located within the at least one predetermined region at the bottom wall,
(f) selecting and picking at least one of the one or more colonies of cells occupying the area or areas determined and recorded in (e), and
(g) inferring that a colony of cells selected and picked in (f) is monoclonal if the position of not less and not more than one cell, as determined and recorded in (c), is located within the area determined and recorded in (e) as the area occupied by the colony of cells selected and picked in (f).

3. A method for picking a monoclonal colony of cells, the method comprising

(a) providing a suspension comprising cells and semi-solid medium in a sample container, the sample container having a substantially flat bottom wall,
(b) centrifuging the sample container such that, after centrifugation, substantially all cells are positioned at the bottom wall,
(c) determining and recording the position of one or more cells located within at least one predetermined region at the bottom wall,
(d) incubating the sample container under conditions suitable for the growth of colonies of cells,
(e) determining and recording the area occupied by one or more colonies of cells, the one or more colonies of cells being located within the at least one predetermined region at the bottom wall,
(f) inferring that a colony of cells is monoclonal if the position of not less and not more than one cell, as determined and recorded in (c), is located within the area determined and recorded in (e) as the area occupied by the colony of cells, and
(g) selecting and picking at least one monoclonal colony of cells.

4. A method for producing a cell line, the method comprising

(a) providing a suspension comprising cells and semi-solid medium in a sample container, the sample container having a substantially flat bottom wall,
(b) centrifuging the sample container such that, after centrifugation, substantially all cells are positioned at the bottom wall,
(c) determining and recording the position of one or more cells located within at least one predetermined region at the bottom wall,
(d) incubating the sample container under conditions suitable for the growth of colonies of cells,
(e) determining and recording the area occupied by one or more colonies of cells, the one or more colonies of cells being located within the at least one predetermined region at the bottom wall,
(f) selecting and picking at least one of the one or more colonies of cells occupying the area or areas determined and recorded in (e), and
(g) producing a cell line by culturing under suitable conditions a colony of cells selected and picked in (f).

5. A method for determining the clonal status of a produced cell line, the method comprising

(a) providing a suspension comprising cells and semi-solid medium in a sample container, the sample container having a substantially flat bottom wall,
(b) centrifuging the sample container such that, after centrifugation, substantially all cells are positioned at the bottom wall,
(c) determining and recording the position of one or more cells located within at least one predetermined region at the bottom wall,
(d) incubating the sample container under conditions suitable for the growth of colonies of cells,
(e) determining and recording the area occupied by one or more colonies of cells, the one or more colonies of cells being located within the at least one predetermined region at the bottom wall,

(f) selecting and picking at least one of the one or more colonies of cells occupying the area or areas determined and recorded in (e),

(g) producing a cell line by culturing under suitable conditions a colony of cells selected and picked in (f), and

(h) inferring that the cell line is monoclonal if the position of not less and not more than one cell, as determined and recorded in (c), is located within the area determined and recorded in (e) as the area occupied by the colony of cells selected and picked in (f) and cultured to establish the cell line in (g).

6. A method for producing a monoclonal cell line, the method comprising

(a) providing a suspension comprising cells and semi-solid medium in a sample container, the sample container having a substantially flat bottom wall,

(b) centrifuging the sample container such that, after centrifugation, substantially all cells are positioned at the bottom wall,

(c) determining and recording the position of one or more cells located within at least one predetermined region at the bottom wall,

(d) incubating the sample container under conditions suitable for the growth of colonies of cells,

(e) determining and recording the area occupied by one or more colonies of cells, the one or more colonies of cells being located within the at least one predetermined region at the bottom wall,

(f) selecting and picking at least one of the one or more colonies of cells occupying the area or areas determined and recorded in (e),

(g) inferring that a colony of cells selected and picked in (f) is monoclonal if the position of not less and not more than one cell, as determined and recorded in (c), is located within the area determined and recorded in (e) as the area occupied by the colony of cells selected and picked in (f), and

(h) producing a monoclonal cell line by culturing under suitable conditions a colony of cells selected and picked in (f) and inferred to be monoclonal in (g).

7. The method according to item 4 or item 5, wherein, in (g), the colony of cells is cultured at 32 - 37 °C and 3 - 12% $CO_2$, preferably 32 - 37 °C and 5 - 10% $CO_2$, more preferably 37 °C and 5 - 7% $CO_2$, in humidified atmosphere in carbonate-buffered growth medium for approximately 3 days to 1 month.

8. The method according to item 6, wherein, in (h), the colony of cells is cultured at 32 - 37 °C and 3 - 12% $CO_2$, preferably 32 - 37 °C and 5 - 10% $CO_2$, more preferably 37 °C and 5 - 7% $CO_2$, in humidified atmosphere in carbonate-buffered growth medium for approximately 3 days to 1 month.

9. The method according to any one of the preceding items, wherein the cells are animal cells, human cells, or insect cells.

10. The method according to any one of the preceding items, wherein the cells are mammalian cells.

11. The method according to any one of the preceding items, wherein the cells are HEK293, CHO, BHK, stem cells, continuous cell lines, primary cells, cancer cell lines, cell lines immortalized by any means, hybridomas of any species, NSO, Sp2/0, HKB11, HuH7, HepG2, SkHep, Vero, Cos, or PerC6, all of them growing adherently, or adapted to growing in suspension and/or growing in suspension.

12. The method according to any one of the preceding items, wherein the substantially flat bottom wall of the sample container is transparent.

13. The method according to any one of the preceding items, wherein, in (c), the position of the one or more cells is determined and recorded by recording a first image of the cells,

wherein a horizontal plane, parallel to the bottom wall and intersecting the cells above the bottom wall, is the focal plane of the first image,

such that the one or more cells located within at least one predetermined region at the bottom wall can be recognized in the first image,

and such that coordinates or any other unique identifier can be assigned to the one or more cells in the first image.

14. The method according to item 13, wherein, in (e), the area occupied by the one or more colonies of cells is determined and recorded by recording a second image of the cells,

wherein a horizontal plane, parallel to the bottom wall and intersecting the cells above the bottom wall, is the focal plane of the second image,

such that the one or more colonies of cells can be recognized in the second image,

and such that coordinates or any other unique identifier can be assigned to the one or more colonies of cells in the first image and these coordinates or unique identifier can be compared to any coordinates or unique identifier assigned to the one or more cells in the first image.

15. The method according to item 14, wherein recording the second image and selecting and picking of a colony of cells is performed using a colony picking apparatus.

16. The method according to item 15, wherein the colony picking apparatus is capable of recording brightfield and fluorescent microscopic images.

17. The method according to any one of items 14 to 16, wherein recording the second image and selecting and picking of a colony of cells is performed using the ClonePix FL system.

18. The method according to any one of items 14 to 16, wherein recording the second image and selecting and picking of a colony of cells is performed using the ClonePix 2 system.

19. The method according to any one of items 14 to 18, wherein, if the method comprises a step of inferring that a colony of cells is monoclonal, a further requirement for inferring that the colony of cells is monoclonal is that the colony of cells has a shape which is indicative of monoclonality.

20. The method according to any one of items 14 to 18, wherein, if the method comprises a step of inferring that a cell line is monoclonal, a further requirement for inferring that the cell line is monoclonal is that the colony of cells cultured to produce the cell line has a shape which is indicative of monoclonality.

21. The method according to any one of the preceding items, wherein the selecting and picking of a colony of cells is based on the presence and intensity of fluorescence of the colony itself and/or directly surrounding the colony, and/or on sufficient distance to the next colony.

22. The method according to any one of items 13 to 21, wherein, during the incubation time in (d), one or more additional image(s) is or are recorded of the cells,

wherein a horizontal plane, parallel to the bottom wall and intersecting the cells above the bottom wall, is the focal plane of the one or more additional image(s),
wherein cells and/or colonies of cells can be recognized in the additional image(s),
and wherein coordinates or any other unique identifier can be assigned to cells and/or colonies of cells in the additional image(s) such that these coordinates or unique identifier can be compared to any coordinates or unique identifier assigned to the one or more cells in the first image.

23. The method according to item 22, wherein the additional images are recorded once a day.

24. The method according to any one of items 13 to 23, wherein the image(s) is or are recorded using light microscopy and a camera.

25. The method according to any one of items 14 to 24, wherein the first image and any additional image(s), but not the second image, is or are recorded using an imaging cytometer.

26. The method according to item 25, wherein the imaging cytometer is capable of recording brightfield microscopic images of the cell culture.

27. The method according to any one of items 14 to 26, wherein the first image and any additional images, but not the second image, is or are recorded using a Celigo cytometer.

28. The method according to any one of the preceding items, wherein, after (b) but before (c), the method further comprises a step of choosing the at least one predetermined region at the bottom wall based on a distribution of cells in the sample container.

29. The method according to item 28, wherein the step of choosing the at least one predetermined region at the bottom wall is carried out with the help of a light microscope or with the help of a light microscope and a computer.

30. The method according to item 29, wherein the light microscope is an imaging cytometer, such as a Celigo cytometer, or a colony picking apparatus, such as a ClonePix FL system or a ClonePix 2 system.

31. The method according to any one of the preceding items, wherein the at least one predetermined region at the bottom wall corresponds to the entire surface area of the bottom wall which is in contact with the suspension.

32. The method according to any one of the preceding items, wherein, in (c), the position of each cell located within the at least one predetermined region at the bottom wall is determined and recorded.

33. The method according to any one of the preceding items, wherein, in (a), a concentration of the cells in the suspension is 250 to 1000 cells/ml if the cells are stable serum-free cell lines, 50 to 500 cells/ml if the cells are stable serum-containing cell lines, 1000 to 5000 cells/ml if the cells are obtained by serum-free transfection, 500 to 2000 cells/ml if the cells are obtained by serum-containing transfection, $10^5$ to $10^6$ cells/ml if the cells are obtained by hybridoma fusion, or 1000 cells/ml if the cells are HEK293 cells.

34. The method according to any one of the preceding items, wherein at least one of the one or more colonies of cells occupying the area or areas determined and recorded in (e) is stained with a contrast enhancing agent in order to assist in selecting and/or picking the colony.

35. The method according to any one of the preceding items, wherein at least one of the one or more colonies of cells occupying the area or areas determined and recorded in (e) is stained with a fluorescent dye in order to assist in selecting and/or picking the colony.

36. The method according to any one of the preceding items, wherein the cells of at least one of the one or more colonies of cells occupying the area or areas determined and recorded in (e) comprise, transcribe, translate, replicate, express, display on their surface, express intracellularly, and/or secrete a biological molecule of interest or a biological entity of interest.

37. The method according to item 36, wherein the biological molecule of interest is selected from the group consisting of: a protein, a polypeptide, a peptide, a nucleic acid, and a part thereof.

38. The method according to item 36, wherein the biological entity of interest is selected from the group consisting of: a virus particle, a viral vector, and a part thereof.

39. The method according to any one of the preceding items, wherein the semi-solid medium is based on methyl cellulose.

40. The method according to any one of the preceding items, wherein the semi-solid medium contains one or more additives selected from the group consisting of: monoclonal or polyclonal antibodies, or fragments thereof, labelled with a fluorescent dye; proteins, such as receptors or ligands, labelled with a fluorescent dye; fluorescent dyes specific for a cellular component such as for nucleic acids; and fluorogenic enzyme substrates or enzyme inhibitors, such as fluorescein isothiocyanate (FITC)-labelled methotrexate (MTX).

41. The method according to any one of the preceding items, wherein the sample container is a 1-well plate or petri dish, 6-well plate, 12-well plate, 24-well plate, 48-well plate, 96-well plate, 384-well plate, or a 1536-well plate.

42. The method according to any one of the preceding items, wherein, in (b), the centrifuging is performed at 1000 x g for 10 minutes.

43. The method according to any one of the preceding items, wherein, after (b), the cells are capable of dividing.

44. The method according to any one of the preceding items, wherein, in (d), the sample container is incubated at 32 - 37 °C and 3 - 12% $CO_2$, preferably 32 - 37 °C and 5 - 10% $CO_2$, more preferably 37 °C and 5 - 7% $CO_2$, in humidified atmosphere for approximately 3 days to 1 month.

The method of the invention allows determining and recording the position of one or more cells located at the bottom wall. This information can subsequently (e.g. before or after colony picking) be used to determine the clonal status of a colony of cells, i.e. to assess whether a given colony has grown from a single cell or from more than one cell, i.e. whether a given colony is monoclonal. Moreover, this information can be used to assess whether a cell line produced by culturing a picked colony of cells is monoclonal, or to produce a cell line by culturing a picked colony whose clonal status has been determined to be monoclonal.

## BRIEF DESCRIPTION OF THE DRAWINGS

**Figure 1:** Celigo image of a HEK293 cell culture in semi-solid medium, recorded directly after seeding but prior to any centrifugation.

**Figure 2:** Celigo image of the same culture as shown in Fig. 1, recorded after a first centrifugation step (see Example 1).

**Figure 3:** Celigo image of the same culture as shown in Fig. 1 and Fig. 2, recorded after a second centrifugation step (see Example 1).

**Figure 4:** Celigo image of the same culture as shown in Fig. 1-3, recorded after a third centrifugation step (see Example 1).

**Figure 5:** Clonepix image of one 6-well captured 14 days after seeding. Colonies selected for picking are numbered.

**Figure 6:** Celigo images of the same 6-well as in Figure 5 captured at day 14 after seeding, recorded before and after colony picking.

**Figure 7:** Coordinates of colonies #1, #2, #9, #12, and #15 as numbered in Figure 5 given by Clonepix (top panel) and Celigo (bottom panel).

**Figure 8:** Celigo images of colonies #1, #2, #9, #12, and #15 captured at day of cell seeding and 14 days after seeding right before Clonepix picking. Colony numberings are as in Figure 5.

**Figure 9:** Celigo images of one cell growing to a colony of cells in semi-solid medium within 14 days (left column), or three cells growing to a colony of cells (right column).

**Figure 10:** Distribution of mono- or poly-clonal origin of 24 colonies grown in semi-solid medium, monitored by Celigo, and determined adequate for picking by Clonepix technology as shown exemplarily in Figures 5 and 6.

## DETAILED DESCRIPTION OF THE INVENTION

The invention is related to a method for picking a colony of cells from semi-solid medium. The method of the invention is characterized in that the use of semi-solid medium is combined with determining and recording the position of cells after seeding in order to allow an assessment of whether or not a given colony subsequently grown in the semi-solid medium is derived from a single cell, i.e. is monoclonal. In the method of the invention, this assessment is facilitated by a centrifugation step after cell seeding.

In known methods, prior to picking a colony of cells which can then be used to establish a cell line, cells are typically seeded either into liquid or semi-solid medium. In these methods, cell seeding constitutes the so-called cloning step, i.e. the step wherein individual cells should be separated from each other in such a fashion that they can give rise to monoclonal colonies of cells. However, ensuring monoclonality based solely on separation of cells during their seeding is not trivial.

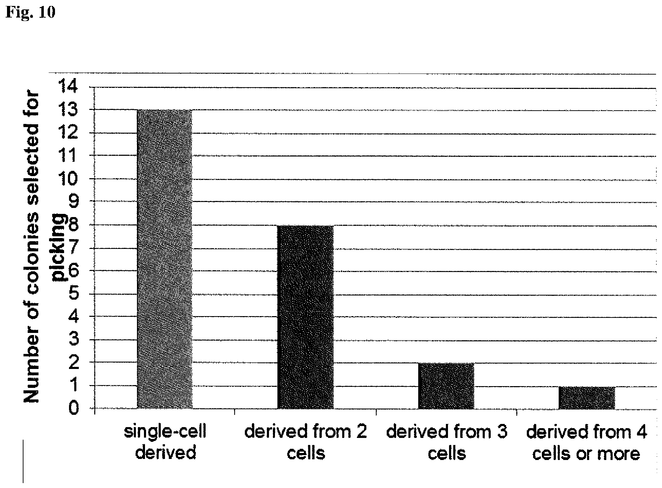

In known colony picking methods relying on semi-solid medium, colonies are typically assessed both for desired cell properties (such as productivity in producing a biological molecule of interest) and for properties thought to correlate with monoclonality (such as colony shape), and then picked on this basis and used to establish cell lines. However, when picking colonies from semi-solid medium, for example using the Clonepix technology, there is currently no direct evidence that a colony identified for picking is derived from a single cell (i.e. that the colony is monoclonal). Probabilities of monoclonality can be estimated based on statistical calculations (and based on empirical studies), but these do not provide direct evidence of monoclonality of a colony. These probabilities can be increased by repeating colony picking in several rounds, but only at the expense of additional time and, even then, it cannot be directly assessed whether a given colony is monoclonal or not. Data presented in the context of the present invention show that the probability of monoclonality can be less than 60% in such semi-solid medium-based colony picking methods (see Example 3 and Figure 10).

Known colony picking methods based on semi-solid medium have not allowed for determining whether a given cell colony is derived from a single cell (i.e. whether the colony is monoclonal). The method of the invention, however, does allow for this verification of monoclonality, namely through a comparison of the position of one or more cells after seeding with the area occupied by a colony grown from at least one of these cells later. This can be achieved, for example, by at least recording a first image just after seeding the cells and a second image before or in the context of selecting and picking a colony. The comparison of these positions and area, e.g. the comparison of at least two images (particularly in the region occupied by a given colony in the second image), reveals whether the colony has arisen from a single cell or not. Namely, it can be assessed how many cells were present (according to their determined and recorded positions, e.g. in the first image) after cell seeding in the area occupied later by a given colony, e.g. in the second image.

In the method of the invention, first, cells are resuspended in semi-solid medium (such as semi-solid medium based on methylcellulose), and the resulting suspension is introduced into a sample container, the sample container having a substantially flat bottom wall in contact with the suspension. Thereby, a suspension comprising cells and semi-solid medium in the sample container is provided for further use in the method of the invention. This step of resuspending cells in semi-solid medium and introducing the suspension into a sample container is also referred to as "cell seeding" or "seeding".

In preferred embodiments of the invention, the semi-solid medium contains one or more additives selected from the group consisting of: monoclonal or polyclonal antibodies, or fragments thereof, labelled with a fluorescent dye; proteins, such as receptors or ligands, labelled with a fluorescent dye; fluorescent dyes specific for a cellular component such as for nucleic acids; and fluorogenic enzyme substrates or enzyme inhibitors, such as fluorescein isothiocyanate (FITC)-labelled methotrexate (MTX). In preferred embodiments of the invention, the sample container is a 1-well plate or petri dish, 6-well plate, 12-well plate, 24-well plate, 48-well plate, 96-well plate, 384-well plate, or a 1536-well plate.

The sample container (e.g. the cell culture dish or plate) containing the suspension of cells and semi-solid medium is then centrifuged such that, after centrifugation, substantially all cells are positioned at the bottom wall of the sample container. Preferably, an ability of cells to divide is retained through the centrifugation step, i.e., after centrifugation, the cells are capable of dividing. Preferably, this centrifuging step is performed on the day of cell seeding, most preferably without delay after cell seeding, i.e. typically within about 30 minutes or at a timepoint well before seeded cells divide, depending on the growth characteristics of the cell type. By contrast, in known protocols for semi-solid medium-based cloning, cells are left undisturbed after seeding in order to allow colonies to form (see, for example, Molecular Devices ClonePix FL Application Guide for New Users, and also Dharshanan S, Hung CS, in Methods Mol Biol. 2014, 1131:105-12: Screening and subcloning of high producer transfectomas using semisolid media and automated colony picker). After centrifugation using appropriate conditions, in the method of the invention, substantially all cells are positioned at the bottom wall of the sample container. Which centrifugation conditions are appropriate for use in the method of the invention depends inter alia on the particular cell type and semi-solid medium used, and appropriate conditions can be determined experimentally (see, for instance, Example 1 herein).

In preferred embodiments of the invention, after centrifugation but before determining the position of one or more cells, at least one predetermined region at the bottom wall is chosen, for example based on a distribution of cells in the sample container. This choosing step may be carried out with the help of a light microscope or with the help of a light microscope and a computer, wherein the light microscope can be, for instance, an imaging cytometer, such as a Celigo cytometer, or a colony picking apparatus, such as a ClonePix FL system or a ClonePix 2 system. However, the at least one predetermined region at the bottom wall may also be chosen by inserting the sample container into a device such an imaging cytometer or a colony picking apparatus in a defined orientation that the device requires, thereby automatically defining at least one predetermined region at the bottom wall of the sample container that will be analyzed by the device.

In the next step of the method of the invention, the position of one or more cells located within at least one predetermined region at the bottom wall is determined and recorded. Preferably, this determining and recording step is performed on the day of seeding the cells, more preferably without delay after centrifugation, e.g. within 30 minutes or at a time before cells divide. Determining and recording the position of the one or more cells can be done, for example, by manual inspection of the sample container using a light microscope or, for example, using a partially or fully automated device such as an imaging cytometer (e.g. a Celigo cytometer (Brooks Life Science Systems, Nexcelom)). In preferred embodiments of the invention, the position of each cell located within the at least one predetermined region at the bottom wall is determined and recorded in this step. Determining and recording the position of one or more cells (e.g. each cell) located within at least one predetermined region at the bottom wall allows later inferring whether or not a colony of cells that has grown within the at least one predetermined region is monoclonal: The colony is monoclonal if the position of not less and not more than one cell, as determined and recorded in the step described in this paragraph, is located within the area occupied by the colony; in that case, the colony is derived from a single cell, i.e. is monoclonal.

In preferred embodiments of the invention, the position of the one or more cells is determined and recorded by recording a first image of the cells, wherein a horizontal plane, parallel to the bottom wall and intersecting the cells above the bottom wall, is the focal plane of the first image, such that the one or more cells located within at least one predetermined region at the bottom wall can be recognized in the first image, and such that coordinates or any other unique identifier can be assigned to the one or more cells in the first image. More preferably, the first image is recorded using an imaging cytometer, most preferably an imaging cytometer capable of recording brightfield microscopic images, for example a Celigo cytometer (Brooks Life Science Systems, Nexcelom).

After determining and recording the position of one or more cells as described above (on the day of seeding), the sample container is incubated under conditions suitable for the growth of colonies. Which conditions are suitable depends inter alia on the type of cells used and, if necessary, suitable conditions can be determined experimentally. In preferred embodiments, during this incubation step, one or more additional image(s) is or are recorded of the cells wherein a horizontal plane, parallel to the bottom wall and intersecting the cells above the bottom wall, is the focal plane of the one or more additional image(s), wherein cells and/or colonies of cells can be recognized in the additional image(s), and wherein coordinates or any other unique identifier can be assigned to cells and/or colonies of cells in the additional image(s) such that these coordinates or unique identifier can be compared to any coordinates or unique identifier assigned to the one or more cells in the first image. Preferably, the additional image(s) is or are recorded using light microscopy and a camera, e.g. an imaging cytometer, more preferably an imaging cytometer capable of recording brightfield microscopic images, for example a Celigo cytometer (Brooks Life Science Systems, Nexcelom).

Then, once one or more colonies of cells have grown, the area occupied by one or more colonies of cells located within the at least one predetermined region at the bottom wall is determined and recorded. Again, this can be done, for example, by manual inspection of the sample container using a light microscope or, for example, using a partially or fully automated device such as a colony picking apparatus (e.g. a Clonepix system such as ClonePix FL or ClonePix 2 (Molecular Devices or Genetix)).

In preferred embodiments of the invention, the area occupied by one or more colonies of cells located within the at least one predetermined region at the bottom wall is determined and recorded by recording a second image of the cells, wherein a horizontal plane, parallel to the bottom wall and intersecting the cells above the bottom wall, is the focal plane of the second image, such that the one or more colonies of cells can be recognized in the second image, and such that coordinates or any other unique identifier can be assigned to the one or more colonies of cells in the second image and these coordinates or unique identifier can be compared to any coordinates or unique identifier assigned to the one or more cells in the first image. More preferably, the second image is recorded using a colony picking apparatus, most preferably a colony picking apparatus capable of recording brightfield and fluorescent microscopic images, such as a ClonePix FL or ClonePix 2 system.

In preferred embodiments of the invention, at least one of the one or more colonies of cells occupying the area or areas determined and recorded as described above is stained with a contrast enhancing agent, such as a fluorescent dye, in order to assist in selecting and/or picking the colony. This may be achieved, for example, by an additive that may be present in the semi-solid medium and that binds to and/or reacts with a biological molecule or entity of interest which the cells of the colony comprise, transcribe, translate, replicate, express, display on their surface, express intracellularly, and/or secrete: Such an additive comprises the contrast enhancing agent, e.g. fluorescent dye, staining the colony. The biological molecule of interest may be selected from the group consisting of: a protein, a polypeptide, a peptide, a nucleic acid, and a part thereof; the biological entity of interest may be selected from the group consisting of: a virus particle, a viral vector, and a part thereof.

Embodiments of the invention relate to a method for picking a colony of cells; in these embodiments, at least one of the one or more colonies of cells whose area was determined and recorded as outlined above is then selected and picked. Preferably, selecting and picking of the at least one colony of cells is performed on the same day as determining and recording the area occupied by the at least one colony of cells. This can be done, for example, by manual inspection of the sample container using a light microscope and manually picking a colony using e.g. a handheld pipette or, for example, this can be done using a partially or fully automated device such as a colony picking apparatus (e.g. a Clonepix system such as ClonePix FL or ClonePix 2 (Molecular Devices or Genetix)). In these embodiments, the fact that the position of one or more cells and the area occupied by one or more colonies of cells within the at least one predetermined region at the bottom wall have been determined and recorded, as described above, allows to compare the recorded position(s) and area(s) at any time before or after selecting and picking of a colony in order to determine whether the colony is monoclonal: The colony is monoclonal if the position of not less and not more than one cell, as determined and recorded after cell seeding and centrifugation, is located within the area occupied by the colony approximately at the time of its selecting and picking; in that case, the colony is derived from a single cell, i.e. it is monoclonal. In preferred embodiments of the invention, the selecting and picking of a colony of cells is based on the presence and intensity of fluorescence of the colony, and/or on sufficient distance to the next colony to avoid picking of an unwanted colony simultaneously. In preferred embodiments of the invention, success of colony picking is visually verified, for example by manually inspecting the remaining cells in the semi-solid medium or, for example, by recording a last image of the remaining cells in the semi-solid medium (the recording being performed in the same fashion as described herein for a first or a second image), after at least one colony has been picked; visually verifying success of colony picking, here, means to assess by eye (manual inspection) or on the last image whether the at least one colony selected for picking was successfully picked.

Embodiments of the invention relate to a method for determining the clonal status of a picked colony of cells, wherein the steps up until and including the selecting and picking of at least one colony of cells are carried out as described above. Subsequently, in these embodiments, it is inferred that a colony of cells selected and picked as described above is monoclonal if the position of not less and not more than one cell, as determined and recorded after cell seeding and centrifugation (see above), is located within the area determined and recorded (as described above) as the area occupied by the colony of cells that has been selected and picked (inferring step). Optionally, in a step of inferring that a colony of cells is monoclonal, a further requirement for inferring that the colony of cells is monoclonal can be that the colony of cells has a shape which is indicative of monoclonality (e.g. colony size, colony roundness, colony roughness, and distance to other colonies).

Embodiments of the invention relate to a method for picking a monoclonal colony of cells; in these embodiments, a step of inferring that a colony of cells is monoclonal (as described above) is carried out after determining and recording the area occupied by one or more colonies of cells but before the step of selecting and picking at least one colony of cells. In these embodiments, rather than selecting and picking any at least one colony of cells (as above), at least one monoclonal colony of cells is picked, i.e. at least one colony is picked that has been inferred to be monoclonal in the above-described inferring step.

Embodiments of the invention relate to a method of producing a cell line. In this method, at least one or more colonies of cells is selected and picked according to the method of the invention for picking a colony of cells, as described above, and then a cell line is produced by culturing under suitable conditions such a selected and picked colony of cells. This method of producing a cell line allows later inferring whether or not a produced cell line is monoclonal: The cell line is monoclonal if the position of not less and not more than one cell (determined and recorded as described above) is located within the area occupied by the colony (determined and recorded as described above) from which the cell line is derived by culturing; in that case, the cell line is derived from a single cell, i.e. is monoclonal.

Embodiments of the invention relate to a method for determining the clonal status of a produced cell line, wherein the steps up until and including the producing of a cell line are carried out as described above. Subsequently, in these embodiments, it is inferred that a produced cell line is monoclonal if the position of not less and not more than one cell, as determined and recorded in a previous step (see above), is located within the area determined and recorded (as described above) as the area occupied by the colony of cells that has been selected, picked and cultured under suitable conditions to produce the cell line.

Embodiments of the invention relate to a method for producing a monoclonal cell line. In these embodiments, a step of inferring that a colony of cells is monoclonal is carried out after the step of selecting and picking at least one colony of cells, as described above. Then, in these embodiments, a monoclonal cell line is produced by culturing under suitable conditions a monoclonal colony of cells.

Alternatively, according to the invention, a method for producing a monoclonal cell line comprises the steps of the above-described method for picking a monoclonal colony of cells and, in addition, a further step of producing the monoclonal cell line by culturing under suitable conditions a monoclonal colony of cells selected and picked as described above.

Moreover, the present disclosure provides a cell line produced by methods described above. In preferred embodiments of the disclosure, the cells of the cell line comprise, transcribe, translate, replicate, express, display on their surface, express intracellularly, and/or secrete a biological molecule of interest or a biological entity of interest; wherein the biological molecule of interest is selected from the group consisting of: a protein, a polypeptide, a peptide, a nucleic acid, and a part thereof; and/or wherein the biological entity of interest is selected from the group consisting of: a virus particle, a viral vector, and a part thereof. Preferred embodiments of the disclosure provide a monoclonal cell line produced by the above-described method of producing a monoclonal cell line. Furthermore, the present disclosure provides a use of the cell line defined in this paragraph, e.g. the monoclonal cell line, in a method for producing the biological molecule and/or the biological entity of interest.

In the methods of the present invention, as outlined above, the centrifugation step facilitates ensuring or verifying monoclonality of a colony (or of a cell line produced by culturing the colony) by allowing a comparison of the position of one or more cells (in one plane) at the bottom wall after seeding and centrifugation with the area occupied by the colony at a later time (before or in the context of selecting and picking a colony). This comparison can be performed, for example, by comparing the first and second images described above showing cells after seeding and centrifugation, and later grown colonies, respectively, in the area occupied by the colony in the second image.

In the case of a comparison based on a first and a second image as described above, the comparison can be performed by comparing the coordinates or any other unique identifier assigned to cells and colonies in the images. In the event that a first and a second image are recorded such that the coordinates or any other unique identifier assigned to cells and colonies cannot be directly compared between the first and second image, the two systems of coordinates or of other unique identifier used in the two images can be harmonized such that the comparison can be drawn. For example, if the point of origin of a (two-dimensional) coordinate system used in the first image lies a certain distance in a certain direction away from the point of origin of a (two-dimensional) coordinate system used in the second image, the coordinates assigned to cells in the first image can be adjusted by this distance in the appropriate direction in order to correspond to coordinates assigned to colonies in the second image, or vice versa.

Preferred embodiments of the invention are related to the development of cell lines using the Clonepix FL platform providing the so-called fluorescence halo picking technique. The Clonepix system consists of a semi-automatic colony picking apparatus which can identify colonies derived from recombinant protein-secreting mammalian cell lines based on their optical properties in semi-solid medium in multi-well plates. Colonies producing a recombinant protein can be identified by adding a fluorochrome-labeled antibody against the secreted recombinant protein in the semi-solid medium which forms an immunoprecipitate around the colony. Thus, a fluorescing halo can be detected surrounding the colony by fluorescence excitation and detection at a specific wavelength. By using imaging software, the size of the fluorescent halo can be measured and related to the colony size. This enables selection of colonies according to their recombinant protein productivity, and picking them - using a steel pin - into multi-well plates. However, the resolution of the optical system of the Clonepix system is not high enough to identify single cells in the semi-solid medium on the day of seeding.

In preferred embodiments of the invention, the inventors instead used the Celigo imaging cytometer to assess whether a given colony is derived from a single cell, and to monitor colony growth in the multi-well plates in semi-solid medium on a regular basis from seeding until picking. The resolution of the Celigo images enabled the inventors to identify single cells, and the software allows tracking of cells over time by saving the coordinates.

In these preferred embodiments, to facilitate imaging of cells growing in semi-solid medium, it was required to centrifuge the culture plates in a laboratory centrifuge at low speed and slow acceleration and high break settings. Cells have to be collected at the bottom of the plate, because the Celigo is able scan a cell culture plate only in one optical plane. By combining both systems (Clonepix and Celigo), as well as centrifugation, the inventors were able to determine whether a given high producer colony picked by Clonepix technology is derived from a single cell.

Thus, e.g. according to these preferred embodiments, the method of the invention makes it possible to grow and pick colonies in and from semi-solid medium, e.g. methylcellulose-containing medium, which are derived from one single cell. Data related to the invention also show that colonies which are derived from more than one cell cannot be reliably distinguished by size or roundness alone from monoclonal colonies. The method of the invention allows tracking and picking of single cell-derived colonies for the development of more stable and uniform cell lines, for example for the production of recombinant proteins for human therapeutic use. The method is applicable for many cell lines, including HEK293, CHO, BHK, stem cells, continuous cell lines, primary cells, cancer cell lines, cell lines immortalized by any means, hybridomas of any species, NSO, Sp2/0, HKB11, HuH7, HepG2, SkHep, Vero, Cos, or PerC6, all of them growing adherently, or adapted to growing in suspension and/or growing in suspension. Preferred embodiments of the invention also offer the advantage that the analyses of images, recorded in order to determine and record the position of one or more cells or the area occupied by a colony, can be done at any time, because all images, and cell and colony positions and areas (e.g. in the form of coordinates), are stored in a computer or on a suitable storage medium using the respective software. This may also facilitate decisions of whether or not a high producer cell line needs to be re-cloned.

In the context of the invention, the term "cells" includes any cell type that can be grown in semi-solid medium. In particular, animal cells, human cells and insect cells are included. In preferred embodiments of the invention, the cells are mammalian cells, such as HEK293, CHO, BHK, stem cells, continuous cell lines, primary cells, cancer cell lines, cell lines immortalized by any means, hybridomas of any species, NSO, Sp2/0, HKB11, HuH7, HepG2, SkHep, Vero, Cos, or PerC6, all of them growing adherently, or adapted to growing in suspension and/or growing in suspension.

In preferred embodiments of the invention, some or all of the cells comprise, transcribe, translate, replicate, express, display on their surface, express intracellularly, and/or secrete a biological molecule of interest or a biological entity of interest. In preferred embodiments of the invention, the biological molecule of interest is selected from the group consisting of: a protein, a polypeptide, a peptide, a nucleic acid, and a part thereof. In preferred embodiments of the invention, the biological entity of interest is selected from the group consisting of: a virus particle, a viral vector, and a part thereof.

The appropriate density at which cells are resuspended in, i.e. seeded into, semi-solid medium in order to provide a "suspension comprising cells and semi-solid medium" depends on the cell type used and can be optimized on a case-by-case basis. A typical cell density is 250 to 1000 cells/ml if the cells are stable serum-free cell lines, 50 to 500 cells/ml if the cells are stable serum-containing cell lines, 1000 to 5000 cells/ml if the cells are obtained by serum-free transfection, 500 to 2000 cells/ml if the cells are obtained by serum-containing transfection, $10^5$ to $10^6$ cells/ml if the cells are obtained by hybridoma fusion, or 1000 cells/ml if the cells are HEK293 cells.

In the context of the invention, the term "semi-solid medium" includes any cell culture medium that has a viscosity appropriate for facilitating spatial separation of individual cells, and of colonies growing therefrom, due to limited diffusion within the medium. In preferred embodiments of the invention, the semi-solid medium is based on methylcellulose, such as CAS number 9004-67-5. In preferred embodiments of the invention, the concentration of methylcellulose in the semi-solid medium is between 1.3 and 2.5 % (see METHODS IN CELL BIOLOGY, Volume 14, David M. Prescott, Academic Press, Jan 13, 1977, pages 240f, and 258f), such as 2% w/v (aqueous solution), and the viscosity is between 1200 cP and 5600 cP, such as 1500 cP or 4000 cP. In preferred embodiments of the invention, the semi-solid medium is Clone-Media (Molecular Devices), e.g. CloneMedia-HEK, CloneMedia-CHO, Clone Media-CHO-G, CloneMedia-G, or Clone-Media. In preferred embodiments of the invention, the semi-solid medium further comprises other additives. In other embodiments, the semi-solid medium is based on agarose.

"Additives" present in the semi-solid medium according to preferred embodiments of the invention include contrast enhancing agents, such as fluorescent agents, that can stain colonies of cells which display a particular property, such as production of a biological molecule of interest or a biological entity of interest. This staining of colonies can then aid in selecting suitable colonies for picking, and picking these colonies. In preferred embodiments of the invention, an additive present in the semi-solid medium is selected from the group consisting of: monoclonal or polyclonal antibodies, or fragments thereof, labelled with a fluorescent dye; proteins, such as receptors or ligands, labelled with a fluorescent dye; fluorescent dyes specific for a cellular component such as for nucleic acids; and fluorogenic enzyme substrates or enzyme inhibitors, such as fluorescein isothiocyanate (FITC)-labelled methotrexate (MTX).

In the context of the invention, the term "sample container having a substantially flat bottom wall" includes any sample container that is, first, suitable for cell culture, and second, has a bottom wall that is substantially flat, preferably such that a centrifugal force perpendicular to the bottom wall (the centrifugal force being appropriate for the centrifuging step as defined in the above-described method of the invention) does not move cells laterally (i.e. in directions perpendicular to the centrifugal force) when these cells are positioned at the bottom wall. In preferred embodiments of the invention, the sample container is a flat-bottom 1-well plate or petri dish, 6-well plate, 12-well plate, 24-well plate, 48-well plate, 96-well plate, 384-well plate, or 1536-well plate, preferably a flat-bottom six-well plate manufactured by Greiner, Corning, CytoOne, Nunc, BD Biosciences, Perkin Elmer, and other manufacturers, as recommended by e.g. by manufacturers of imaging cytometers (such as Celigo) and of colony picking apparatuses (such as ClonePix), for example the 6-Well Greiner™ 657160 Plate. Plates configured to be used with Clonepix FL are also Genetix 6-well black, Genetix Petriwell-1 Plate, Genetix PetriWell-6 Plate, and Nunc Omni Tray. For Clonepix, users can also request to be informed whether a certain plate is adequate or already programmed, via an online form "plate certification", http://mdc.custhelp.com/app/ask. In preferred embodiments of the invention, the substantially flat bottom wall of the sample container is transparent.

In the context of the invention, centrifuging of the sample container is performed using a suitable laboratory centrifuge, such as an Eppendorf 5810R centrifuge. Appropriate conditions for centrifuging the sample container are characterized in that, after centrifugation, substantially all cells in the suspension contained in the sample container are positioned at the bottom wall of the sample container. The particular conditions depend *inter alia* on the particular composition of semi-solid medium and on the particular cell type employed. The appropriate conditions can be determined e.g. by applying a range of conditions in parallel or subsequent test centrifugations, determining by light microscopy whether substantially all cells are positioned at the bottom wall of the sample container, and choosing the minimum centrifugation force and time required for substantially all cells to be so positioned. In preferred embodiments of the invention, centrifugation is performed at 1000 x g for 10 minutes, using an acceleration time of level 2 on a scale from 0 to 9, where 9 is the fastest, and 2 equals 170 seconds (or an angular acceleration of 37 rad/s$^2$) and a deceleration time of level 9 on a scale from 0 to 9, where 9 is the fastest equalling approximately 30 seconds (or an angular deceleration of 209 rad/s$^2$), when using an Eppendorf 5810R centrifuge with an A-4-81 rotor and MTP/flex buckets. Alternatively, 100 s of acceleration time (or 63 rad/s$^2$ angular acceleration) may be used.

In the context of the invention, the phrase "substantially all cells are positioned at the bottom wall" refers to a situation in which, when a microscopic image is recorded of cells at the bottom wall (wherein a horizontal plane, parallel to the bottom wall and intersecting the cells above the bottom wall, is the focal plane of the image), all cells that are visible in the image are also in the focus of the image, i.e. no cells appear out of focus. In such a situation, cells visible in the focus of the image may be located directly at the bottom wall (i.e. touching the bottom wall) and/or directly above the bottom wall (e.g. not touching the bottom wall but so close to it that the cells are still in the focus of the image), which is referred to herein using the general phrase of the cells being "positioned at the bottom wall".

In the context of the invention, the term "determining and recording the position of one or more cells located within at least one predetermined region at the bottom wall" refers to a process of determining the position of one or more such cells relative to a given point of reference and recording this position or these positions as information such that this information can be accessed again at a later time. Preferably, the position of one or more cells is determined and recorded in a fashion wherein this position or these positions is or are defined in two dimensions, e.g. in a plane parallel to the bottom wall of the sample container. As a particularly simple example, the position of one or more such cells can be determined by inspection of the sample container using a light microscope; the position of one or more such cells can be manually determined by adjusting the focus of the microscope to a plane parallel to the bottom wall and intersecting the cells above the bottom wall, such that the cells positioned at the bottom wall can be seen in focus; the position of one or more (for example: all) such cells can then be recorded, for example by manually applying markings to the sample container (e.g. with a permanent marker, on the outside surface of the bottom wall of the sample container). In preferred embodiments of the invention, the position of one or more such cells is determined and recorded by analyzing the sample container using an imaging cytometer, such as a Celigo cytometer, that records a first image of the cells and assigns coordinates or any other unique identifier to the one or more cells in the first image.

The term "imaging cytometer", as used herein in describing preferred embodiments of the invention, refers to a device capable of analyzing properties of cells that are positioned in one plane, such as cells that are positioned at the bottom wall of the sample container according to the present invention, or such as adherent cells adhering to the bottom wall of a flat-bottom sample container. Typically, such an imaging cytometer analyzes cells by recording one or more microscopic images of the cells and then performing analyses on these images. For instance, coordinates or any other unique identifier may be assigned to one or more cells visible in the recorded image(s) and saved to a computer or suitable storage medium, thereby determining and recording their positions. An example of an imaging cytometer is a Celigo cytometer (Brooks Life Science Systems, Nexcelom).

When describing the recording of images in preferred embodiments of the invention above, the term "plane" is used to refer to an optical plane, i.e. a two-dimensional plane in three-dimensional space. The plane which an image focuses on is referred to as the "focal plane" of the image.

In the context of the invention, the term "predetermined region at the bottom wall" refers to all or part of the surface area of the bottom wall that is in contact with the suspension comprising cells and semi-solid medium. In embodiments of the invention, a predetermined region corresponds to the entire surface area which is in contact with the suspension. In other embodiments of the invention, at least one predetermined region at the bottom wall that corresponds to part or all of the surface area in contact with the suspension is chosen after centrifuging the sample container but before determining and recording the position of one or more cells located within the at least on predetermined region; preferably, the choosing of the at least one predetermined region is based on a distribution of cells in the sample container; for example, at least one predetermined region may be chosen on the basis of a minimum distance between cells located within the at least one predetermined region.

In preferred embodiments of the invention, images are recorded using light microscopy and a camera. Recording a first image does not necessarily mean that only one image is recorded but, rather, that one or more images are recorded and one image is chosen as the first image. This also applies to other images recorded according to embodiments of the present invention. The image chosen as the first image is one wherein individual cells can be recognized and wherein coordinates can be assigned to individual cells, preferably for matching to the coordinates of colonies shown in other images recorded later, such as a second image (see below). In preferred embodiments of the invention, the first image is recorded using an imaging cytometer, such as an imaging cytometer capable of capturing brightfield microscopic images of the cell culture, such as a Celigo cytometer (Brooks Life Science Systems, Nexcelom). The first image recorded in preferred embodiments of the invention is recorded from above or below the sample container, and the image focuses on a horizontal optical plane (i.e. an optical plane that is parallel to the substantially flat bottom wall of the sample container) intersecting the cells above the bottom wall of the sample container.

In preferred embodiments of the invention, a first image is recorded without delay after the centrifuging step, i.e. typically within 30 minutes or at a timepoint well before seeded cells divide, depending on the growth characteristics of the cell type.

In preferred embodiments of the invention, during the phase of colony growth between determining and recording the position of one or more cells and determining and recording the area occupied by one or more colonies, respectively, the sample container, which contains the suspension, is incubated at 32 - 37 °C and 3 - 12% $CO_2$, preferably 32 - 37 °C and 5 - 10% $CO_2$, more preferably 37 °C and 5 - 7% $CO_2$, in humidified atmosphere for approximately 3 days to 1 month. These conditions are examples of "conditions suitable for the growth of colonies of cells".

During the phase of colony growth, colonies of cells may grow while cells adhere to the bottom wall and/or while cells are suspended in the semi-solid medium directly above the bottom wall. These growth forms can be distinguished microscopically, as cells display different morphologies depending on whether they adhere to the bottom wall or not.

In the context of the invention, the term "colony" refers to a group or aggregation of cells in semi-solid medium (i.e. at least two cells touching each other). Such a colony typically grows during the incubation period following the steps of seeding of the cells into semi-solid medium, centrifugation, and determining and recording the position of one or more cells. In principle, a colony may grow either from a single cell sufficiently far removed from all other cells in the semi-solid medium for these other cells not to form part of the colony grown from the single cell, or a colony may grow from several cells close enough to each other in the semi-solid medium for more than one of them to form part of the colony grown from them.

In the context of the invention, the term "determining and recording the area occupied by one or more colonies of cells" refers to a process of determining the area occupied by one or more colonies of cells relative to a given point of reference and recording this area or these areas as information such that this information can be accessed again at a later time. As a particularly simple example, the areas of one or more colonies can be determined by inspection of the sample container using a light microscope; the area of one or more such cells can be manually determined by adjusting the focus of the microscope to a plane parallel to the bottom wall and intersecting the cells above the bottom wall, such that the colonies of cells located at the bottom wall can be seen in focus; the areas occupied by one or more such colonies can then be recorded, for example by manually applying markings to the sample container (e.g. with a permanent marker, on the outside surface of the bottom wall of the sample container) or by committing these areas to memory. In preferred embodiments of the invention, the position of one or more such colonies of cells is determined and recorded by analyzing the sample container using a colony picking apparatus, such as a ClonePix system (e.g. a ClonePix FL or ClonePix 2 system), that records a second image of the cells and assigns coordinates or any other unique identifier to the one or more colonies of cells in the first image. Therefore, the area occupied by one or more colonies of cells at the bottom wall may be determined and recorded as an area or areas in a two-dimensional coordinate system, for example. The area or areas thus determined and recorded, in preferred embodiments of the invention, are amenable to comparison with the position of one or more cells previously determined and recorded; in particular, it can be analyzed whether not less and not more than one cell was positioned (after centrifugation) in the area occupied by a given colony, which allows inferring whether the given colony is monoclonal, i.e. derived from a single cell.

The term "colony picking apparatus", as used herein in describing preferred embodiments of the invention, refers to a device capable of selecting and picking at least one colony of cells from semi-solid medium in a sample container. Preferably, the selecting and picking of colonies is partially or fully automated in such a device. Preferably, a colony picking apparatus is capable of recording brightfield and fluorescent microscopic images of cells and colonies of cells. In preferred embodiments of the invention, based on properties of colonies visible on such images, the device can (e.g. with input from the user or according to predetermined rules) select and pick colonies of cells. An example of a colony picking apparatus is a ClonePix system, such as a ClonePix FL or ClonePix 2 system (Molecular Devices, Genetix).

In the context of the invention, "selecting a colony of cells" means identifying a colony as adequate for picking based one or more criteria such as colony size, colony roundness, colony roughness, and distance to other colonies (e.g. a minimum distance between colonies, such as 0.9 mm, may be chosen as one of the one or more criteria that need(s) to be met in order for a colony to be identified as adequate for picking, i.e. for a colony to be selected). In preferred embodiments of the invention, such as embodiments wherein a colony picking apparatus (such as a Clonepix system) is used for selecting a colony of cells, the one or more criteria on the basis of which a colony is selected as adequate for picking may include the intensity of fluorescence (or the intensity of another type of staining) of the colony itself and/or directly surrounding the colony like a fluorescent "halo", for example as an immunoprecipitate. This fluorescence intensity may result from detection of a biological molecule or entity of interest secreted by cells of the colony or displayed on their surface, for example, detection being achieved by a fluorescently labelled additive in the semi-solid medium.

In the context of the invention, the term "picking a colony of cells" refers to the process of transferring one or more cells of a colony, but not any cells that do not form part of the colony, from the sample container that the colony has grown in to another sample container. Alternatively, instead of transferring to another sample container, the one or more cells of a colony may be used in another method (e.g. a method of analysis or a method of production) such as polymerase chain reaction (PCR), fluorescence-activated cell sorting (FACS) or cell cultivation. In preferred embodiments of the invention, during picking a colony, one or more cells of the colony are transferred to a well of a multi-well plate (such as a 96- or 384-well plate) containing cell culture medium suitable for cultivation of the type of cells that the colony is made up of. In preferred embodiments of the invention, during picking a colony, the one or more cells of the colony are aspirated out of the semi-solid medium using a pipette tip or any similar device (e.g. a stainless steel needle such as the one that forms part of a colony picking apparatus (such as a ClonePix FL or ClonePix 2 system)) and introduced into liquid growth medium by pushing the colony out of the pipette tip or similar device using compressed air and flushing the pipette tip or similar device.

In preferred embodiments of the invention, the colony is picked based on the presence and intensity of its fluorescence, a technique also known as "fluorescent halo picking technique". In such embodiments, an appropriate additive is present in the semi-solid medium to fluorescently stain colonies which comprise, transcribe, translate, replicate, express, display on their surface, express intracellularly, and/or secrete a biological molecule of interest or biological entity of interest able to bind to or react with the additive.

In the preferred embodiments of the invention, the first and the second images are compared in the area occupied by a given colony in the second image, wherein the colony of cells is derived from a single cell if the same area in the first image contains only one cell. This comparison enables an assessment of whether or not a given colony has arisen from a single cell.

In the context of the invention, the term "clonal status" refers to a property of a colony of cells or of a cell line, namely to the property of being monoclonal or not.

In the context of the invention, the term "monoclonal", used e.g. to characterize a colony of cells or a cell line, means "derived from a single cell". In other words, a monoclonal colony is a colony that has grown from a single cell, and a monoclonal cell line is a cell line that has grown from a single cell.

In preferred embodiments of the invention, colonies of interest are identified by comparing a first image recorded using a Celigo cytometer (showing one or more cells located within at least one predetermined region at the bottom wall) with a second image recorded by the ClonePix FL (showing picked colonies, or colonies to be picked), wherein one or more cells and one or more colonies have been assigned coordinates to identify their position and areas occupied, respectively. Individual cells and/or colonies are thus identified by their coordinates given by the Celigo or ClonePix systems, although different (two-dimensional) coordinate systems are used. The ClonePix FL has its point of origin in the upper left corner of the plate whereas the Celigo has its point of origin in the middle of each well, but both systems measure in $\mu$m or mm and are thus comparable. In preferred embodiments of the invention, Celigo coordinates are read by using the software mode "tumorsphere detection". In the "gate" sub-menu in the tab "classes", the population details including the coordinates of a selected colony can be displayed.

In preferred embodiments of the invention, the comparison between images recorded using a Celigo cytometer and images recorded using the ClonePix FL system is achieved through a method to harmonize cell and colony coordinates from both systems, enabling cell and colony tracking, as described in the following:

The Clonepix coordinate system has its origin (x=0, y=0) in the upper left corner of the plate.

The Celigo coordinate system has its origin in the center of each well. Therefore, the Clonepix coordinates of each well center have to be determined either experimentally by using plates whose well centers are marked, or by using coordinates given by the plate supplier; e.g. Greiner delivers well center coordinates.

The coordinates (x and y values) of every picked or photographed cell or colony of cells are given as information by the respective Clonepix or Celigo software.

Celigo and Clonepix coordinates can be related to each other by using the following equations:

| | |
|---|---|
| $x_{Celigo}$ | x-coordinate measured by Celigo [$\mu$m] |
| $x_{Clonepix}$ | x-coordinate measured by Clonepix [mm] |
| $x0_{Clonepix}$ | center coordinate of a well stored in Clonepix software [mm] |
| $y_{Celigo}$ | y-coordinate measured by Celigo [$\mu$m] |
| $y_{Clonepix}$ | y-coordinate measured by Clonepix [mm] |

14

(continued)

y0$_{\text{Clonepix}}$    center coordinate of a well stored in Clonepix software [mm]

$$x_{\text{Celigo}} = (x_{\text{Clonepix}} - x0_{\text{Clonepix}}) * 1000$$

$$y_{\text{Celigo}} = (y_{\text{Clonepix}} - y0_{\text{Clonepix}}) * 1000$$

EXAMPLES

*Example 1: Determining appropriate centrifugation conditions*

Appropriate centrifugation conditions for use in the method of the invention, namely in the centrifuging step following seeding of cells into semi-solid medium (for substantially all cells to be positioned at the bottom wall of the sample container after centrifugation), depend inter alia on the particular cell type and type of semi-solid medium used. Appropriate conditions can be determined experimentally on a case-by-case basis, as exemplified below for HEK293 cells.

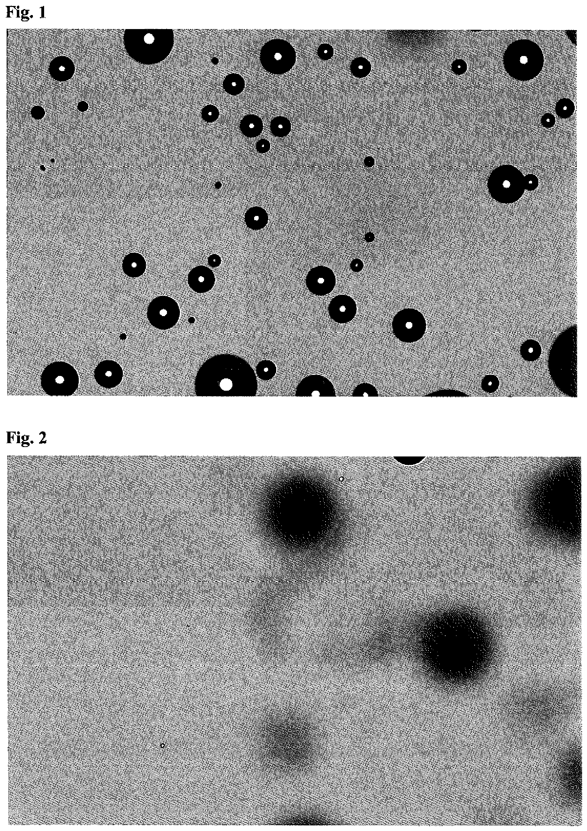

HEK293 cells were resuspended at a density of 1000 cells/ml in semi-solid medium (90% v/v CloneMedia-HEK (Molecular Devices, order K8685); 10% v/v CD293 medium (Life Technologies) supplemented with 4 mM L-glutamine (Life Technologies), 10 mM HEPES (Life Technologies), and 15 µM phenol red), according to the manufacturer's instructions (Molecular Devices). 2 ml of this cell suspension was added into one well of a 6-well plate (Greiner). An image was recorded of the well containing the cell suspension (Fig. 1), but no suitable focus could be found due to the distribution of cells in three dimensions. Then, the plate was subjected to several centrifugation steps in an Eppendorf 5810R centrifuge with an A-4-81 rotor and MTP/flex buckets at 1000 x g using very low acceleration (2 of 9) and high brake (9 of 9) force, and an image was recorded after each centrifugation step, in order to determine how long the centrifugation needed to be in order for substantially all cells to arrive at the bottom wall of the plate, i.e. in one optical plane.

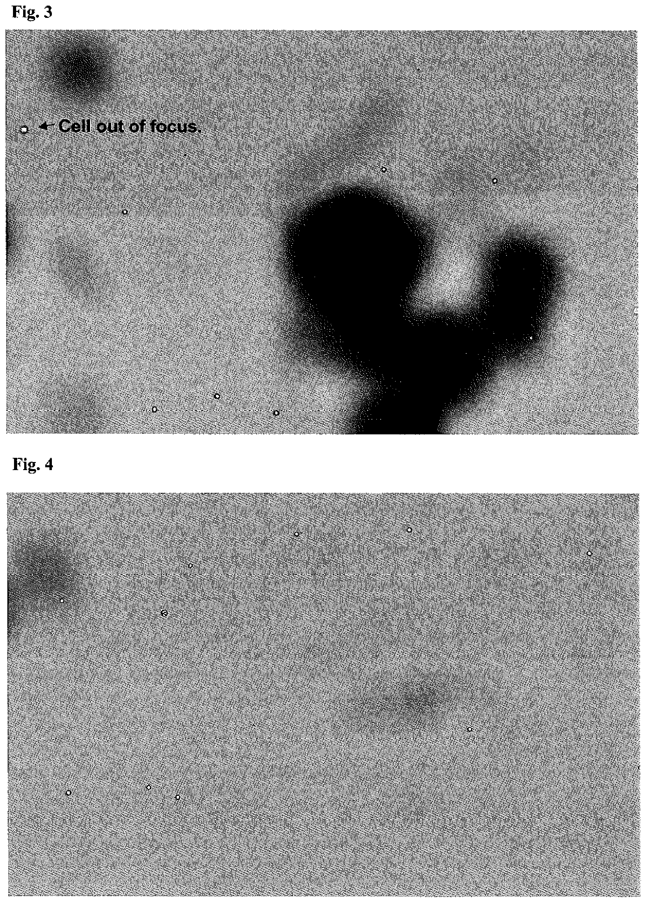

After a first centrifugation step of one minute at approximately 400 x g (settings 1000 x g, acceleration 2 [195 sec according to centrifuge specifications, but in our experience only 100 sec; may vary considerably], brake 9 [30 sec]); the run was stopped after one minute, therefore 400 x g were reached), an image was recorded (Fig. 2) showing the same section as Fig. 1, but no suitable focus could be found to capture all cells in one optical plane, however, some cells were already found at the bottom of the well. After an additional centrifugation step for 1 min 50sec (10 sec at 1000g effectively, acceleration 2, brake 9) another image was recorded (Fig. 3), where still some cells were not in focus. However, after a final centrifugation step of five minutes (3 min 20 sec at 1000g, acceleration 2, brake 9), a final image could be recorded showing only cells in focus (Fig. 4).

In contrast, merely incubating a 6-well plate containing the suspension of cells and semi-solid medium for 5.5 hours at 37 °C and 5 - 7% $CO_2$ in humidified atmosphere, without any centrifugation, did not allow for cells to settle into one plane. This result illustrates that, following known protocols for cell line development using semi-solid medium, such as the protocols for ClonePix platforms (Molecular Devices), i.e. without centrifugation (leaving the cells "undisturbed" after seeding), it is not possible to record a meaningful image of the cells on the day of seeding. Therefore, following such known protocols, monoclonality of a given colony cannot be verified as it can be using the method of the invention, namely based on an image of the cells (in one plane) recorded on the day of seeding.

*Example 2: Comparing coordinates of colonies on images recorded using Celigo and ClonePix FL, respectively*

A 6-well plate containing colonies derived from HEK293 cells grown in semi solid medium was imaged by the Celigo using the "tumorsphere 1" application to provide images for comparison of the coordinates of the systems Celigo and ClonePix FL. The imaging options used with Celigo were the following: Application Name: Tumorsphere1 or Target 1; Plate: Greiner 657160 Plate; Exposure: about 1800 µs brightfield; percent well mask size: 99; algorithm: 0; intensity threshold: 10-50; precision: 2; filter size: 12-50; focus position A: 3.3; focus position B: 3.3; additional information see files "Well level Data xxx"

After having inserted the 6-well plate into the Clonepix FL system, imaging was initiated in the respective system software. A "Preview Picture" was taken and the average colony diameter was fixed (eg. 0.46 mm) as well as imaging options white light 100ms, 2LED brightness / FITC: 1000ms, 128 LED brightness and algorithm local threshold. Next the wells of interest were selected, and images were taken as preset. In the tab "Graph" in the submenu "Groups" definitions for clones of interest were made. (eg. "too big" more than > 0.7 mm; "too small" equal or less than 0.06mm, proximity not less than 0.9 mm; compactness (defined as 0= not compact, 1= perfect circle) not less than 0.6; axis ratio (defined as ratio of the minimum and maximum radiuses of the feature) not less than 0.6; exterior mean intensity FITC more than 23.55 and not more than 500). Accepted clones were highlighted green automatically, and were picked in the next step into 96 well plates.

Using the option "review results" the statistic parameters of all clones were exported as *.csv file and moved to MS Excel containing x and y coordinates of the clones given in mm. Using the Celigo Software in the Tab "Results" the colony of interest was zoomed in as near as possible in the picture taken before picking. Without changing the image section on the screen the scan from day 0 was loaded by changing the date in the scan menu appropriately to reassess the presence of only one cell in the defined area of the colony.

The coordinates of the selected colony could be obtained by using the menu "Gate". First, one had to click on the well of interest of the plate. The colony of interest was found by comparing the "clone pattern". It was zoomed in as near as possible, and ensured that the option "graphic overlay" was turned on and changed to submenu "Classes". The colony was bordered in a colour of choice. The colony was selected by left-click, and its coordinates read in the field "object level data".

The coordinates of 5 colonies in well A2 of a 6-well plate as measured by Celigo or their theoretical Celigo coordinates calculated from the Clonepix data (using the above-described method of harmonizing cell and colony coordinates from Celigo and Clonepix systems, i.e. by relating coordinates from both systems to each other using the above-described equation) were compared, and the results of this comparison are presented in Table 1 below:

**Table 1**

| Colony number | Clonepix coordinates | | Celigo coordinates | | Center coordinates for well A2 as measured by Clonepix | | Theoretical Celigo coordinates calculated from Clonepix coordinates | | Measured minus calculated Celigo coordinates | |
|---|---|---|---|---|---|---|---|---|---|---|
| | x [mm] | y [mm] | x [$\mu$m] | y [$\mu$m] | x0 [mm] | y0 [mm] | x [$\mu$m] | y [$\mu$m] | x [$\mu$m] | y [$\mu$m] |
| #1 | 68.53 | 31.76 | 4498 | 8250 | 63.88 | 23.24 | 4649 | 8518 | -151 | -268 |
| #2 | 72.54 | 23.03 | 8513 | -468 | 63.88 | 23.24 | 8655 | -206 | -142 | -262 |
| #9 | 71.29 | 16.03 | 7252 | -7441 | 63.88 | 23.24 | 7412 | -7215 | -160 | -226 |
| #12 | 53.30 | 28.01 | -10765 | 4579 | 63.88 | 23.24 | -10583 | 4771 | -182 | -192 |
| #15 | 50.11 | 18.89 | -13983 | -4632 | 63.88 | 23.24 | -13766 | -4346 | -217 | -286 |
| Mean deviation of calculated versus measured Celigo coordinates | | | | | | | | | **-170** | **-247** |

It can be concluded from the data in Table 1 that a single cell or a colony on a Celigo image can be uniquely identified based on Clonepix data, because the maximum proximity of 900 $\mu$m of one colony to another, which is predefined as a requirement to select a colony adequate for picking by Clonepix, is much higher than the observed deviations in x and y directions (170 and 247 $\mu$m, respectively).

***Example 3: Colony picking and monoclonality verification using the method of the invention***

***Introduction***

This example relates to the development of cell lines using the Clonepix FL platform, which provides the so-called fluorescence halo picking technique, in combination with the Celigo cytometer and a centrifugation step, according to the method of the invention.

***Methods and Materials***

**Cell culture**

HEK293H cells (Life Technologies) secreting a recombinant protein were generated by transfection of a mammalian expression plasmid vector based on pBudCE4.1 (Life Technologies catalog no. V532-20) by nucleofector technology encoding the gene of interest (coding for a human IgG fusion protein), and stably transfected cells were selected by resistance to Zeocin added at 10 μg/ml to the culture medium (CD293 medium (Life Technologies) supplemented with 4 mM L-glutamine (Life Technologies), 10 mM HEPES (Life Technologies), 5 μg/ml insulin (Lonza), and 2 g/l soy peptone (Baxter preparation)) caused by co-expression of a resistance gene also encoded on the same plasmid. Cell pools after antibiotic selection were seeded into semi-solid medium (Clone Media HEK, Molecular Devices, catalog no. K8685) in 6-well plates, and a polyclonal anti human IgG antibody labeled with FITC was added at a concentration of 5 μg/mL. From the semi-solid medium, colonies were picked into the same culture medium as above, optionally supplemented with 1 part of 3 conditioned supernatant in the medium to facilitate growth at low cell density and after cloning and picking stress. The conditioned supernatant can be obtained by high-speed centrifugation of an aliquot of the culture supernatant of the selected cell pool after two to three culture days. Clones were further expanded for characterization.

**Celigo imaging**

The 6-well plates containing cell colonies in semi-solid medium were centrifuged in an Eppendorf 5810R centrifuge at 1000 x g for 10 minutes at very low acceleration (2 of 9) and high brake (9 of 9) to enable cells or colonies to move to the bottom wall of the plate for imaging. Images were captured using a Celigo cytometer on a regular basis until colony picking. It was required to collect substantially all cells at the same level at the bottom of the plate, because the Celigo cytometer can only scan a whole cell culture plate within the same optical plane or z-coordinate value. Imaging settings were adjusted to taking 276 pictures per 6-well plate within approximately 5 minutes. In detail, cell (growth) tracing was achieved by creating a new Plate ID in the Celigo software. Thus, all taken photographs of the plate were filed as one experiment containing all performed scans of one plate at given times (essentially directly after plating and directly before picking, optionally several times in between). The pictures were linked to each other, enabling to switch from the colony of interest on the day of picking to the very same position directly after plating to verify monoclonality of the colony.

**Clonepix colony picking**

Clones were picked using the ClonePix FL 14 days after splitting the cells into semisolid medium. For picking the ClonePix FL was prepared using the program "prepare for pickrun". (Alignment of the pins; disinfection with PERA Safe and UV for 10 minutes, cleaning with aqua dest.). For picking the program "PICKRUN" was used. According to the menu the parameters were chosen (eg. plate type Greiner 6 well, Acquisition Options Trans WL and FITC; Prime configuration Trans WL; destination Wells).

After having inserted the 6-well plate into the Clonepix FL system, imaging was initiated in the respective system software. Colonies were selected based on morphological features measured in the white light channel, and fluorescing halo size and intensity as measured in the fluorescence channel. A "Preview Picture" was taken and the average colony diameter was fixed (eg. 0.46 mm) as well as imaging options white light 100ms, 2LED brightness / FITC: 1000ms, 128 LED brightness and algorithm local threshold. Next the wells of interest were selected, and images were taken as preset. In the tab "Graph" in the submenu "Groups" definitions for clones of interest were made. (eg. "too big" more than > 0.7 mm; "too small" equal or less than 0.06mm, proximity not less than 0.9 mm; compactness (defined as 0= not compact, 1= perfect circle) not less than 0.6; axis ratio (defined as ratio of the minimum and maximum radiuses of the feature) not less than 0.6; exterior mean intensity FITC more than 23.55 and not more than 500). Accepted clones were highlighted green automatically, and were picked in the next step into 96 well plates.

Using the option "review results" the statistic parameters of all clones were exported as .csv file and moved to MS Excel containing x and y coordinates of the clones given in mm. Using the Celigo Software in the Tab "Results" the colony of interest was zoomed in as near as possible in the picture taken before picking. Without changing the image section on the screen the scan from day 0 was loaded by changing the date in the scan menu appropriately to reassess the presence of only one cell in the defined area of the colony.

The coordinates of the selected colony could be obtained by using the menu "Gate". First, one had to click on the well of interest of the plate. The colony of interest was found by comparing the "clone pattern". It was zoomed in as near as possible, and ensured that the option "graphic overlay" was turned on and changed to submenu "Classes". The colony was bordered in a colour of choice. The colony was selected by left-click, and its coordinates read in the field "object level data".

**Colony and cell identification based on Celigo and Clonepix coordinates**

The colonies of interest were identified by comparing the Celigo images with an image taken by the ClonePix FL showing picked colonies marked up. Colonies were also identified by their coordinates given by the Celigo or Clonepix systems, although different coordinate systems are used (see above description of how to harmonize, i.e. relate to each other, the coordinates of the two different systems). The ClonePix FL has its point of origin in the upper left corner of the plate whereas the Celigo has its point of origin in the middle of each well, but both systems measure in [μm] or [mm]. Celigo coordinates are read by using the software mode "tumorsphere detection". In the "gate" sub-menu in the tab "classes", the population details including the coordinates of a selected colony or cell can be displayed. All Celigo images taken during the time period from seeding into semi-solid medium until colony picking and one image after picking were used for assessing monoclonality and growth of the picked colonies.

### *Results and Conclusion*

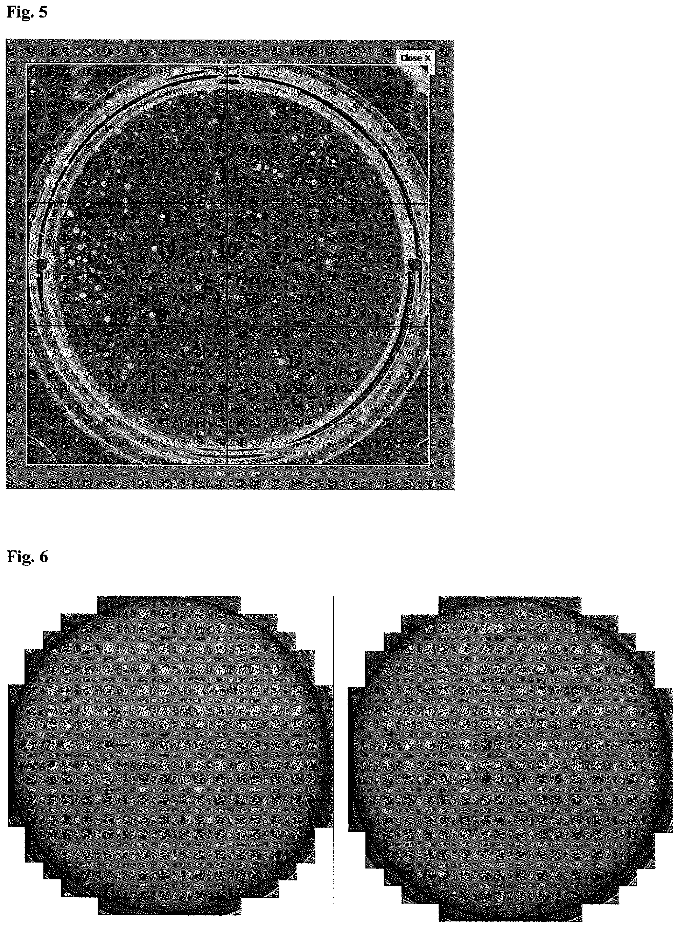

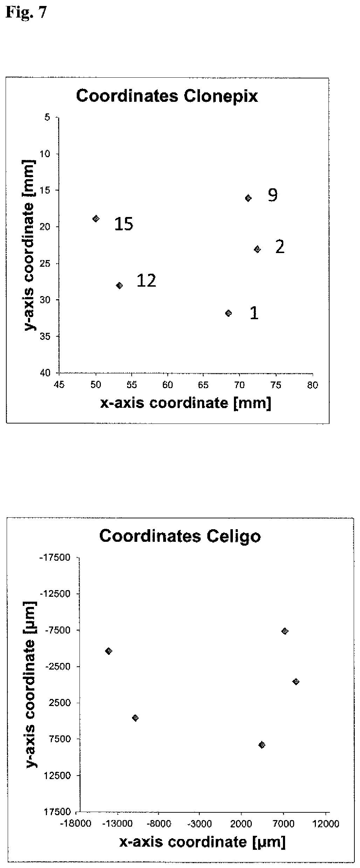

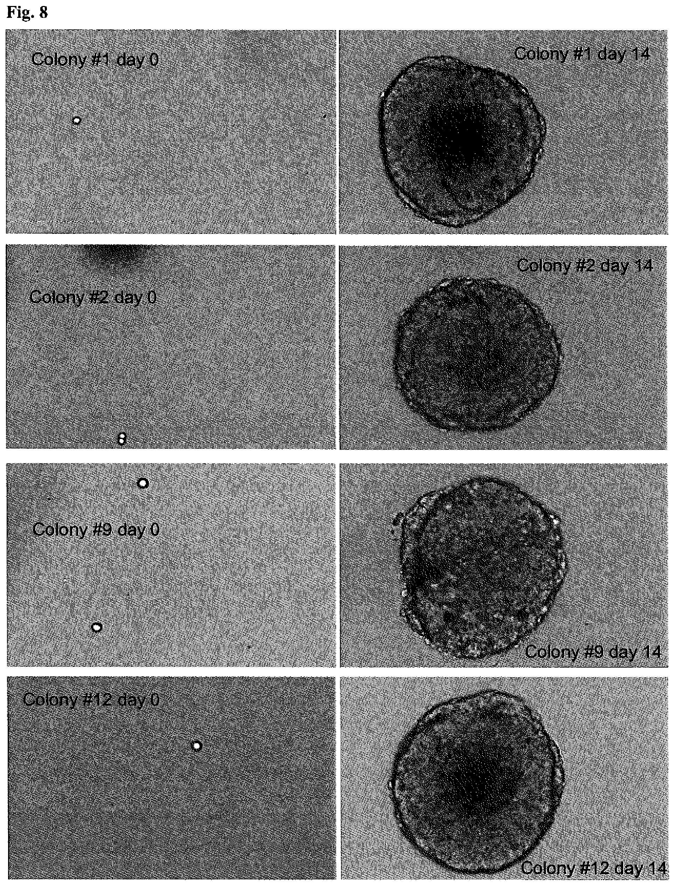

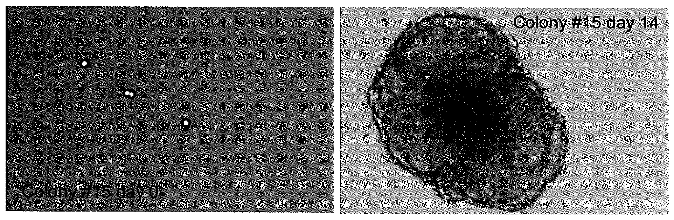

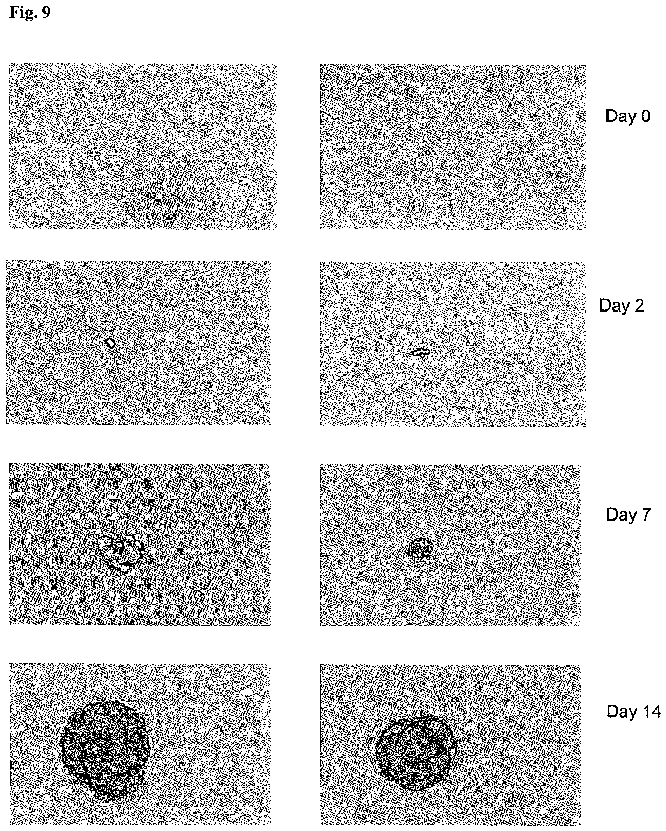

In a representative picking experiment, 250 cells per mL were seeded into a six well plate, and images were captured periodically by the Celigo cytometer. After 14 days, the plates were scanned in the Clonepix instrument, colonies were selected for picking, and picked into 96-well plates. In Figure 5, the Clonepix image of one representative well can be seen showing 15 colonies numbered and selected for picking. Figure 6 shows the corresponding Celigo images captured before and after Clonepix picking with the 15 colonies marked by circles. Figure 7 shows the colony coordinates of 5 selected colonies as given by Clonepix and Celigo technology demonstrating that coordinates from one system can be used to locate the colonies in the other system. In Figure 8, Celigo images of these 5 colonies at higher magnification captured on day zero and day 14 are shown demonstrating tracking of colony growth from the beginning. Colonies derived from 1, 2 and more cells can be clearly identified. In Figure 9, the history of one single-cell derived colony, i.e. monoclonal colony, and one colony derived from 3 cells is compared by showing Celigo images captured on days 0, 2, 7 and 14 after cell seeding. It can be seen, that after 2 days, 2 or 6 cells are visible, respectively, in accordance with approximately 24-36 hours doubling time of HEK293 cells. In Figure 10, the distribution of mono- or poly-clonal origin of 24 colonies grown in semi-solid medium, monitored by Celigo, and selected for picking by Clonepix technology is shown for this experiment. It can be concluded, that the probability that a colony picked by Clonepix technology is not derived from a single cell can be as high as approximately 45 %, but also that, in contrast to known methods, the method of the invention allows determining whether or not a given colony is derived from a single cell.

In summary, it is shown here for the first time that it is possible to grow and pick colonies in and from semi-solid, methylcellulose containing medium, which are derived from one cell, i.e. which are monoclonal.

It is also shown here that colonies which are derived from more than one cell cannot be reliably distinguished from monoclonal colonies by size or roundness, for example. The method used here, in contrast, allows tracking and picking of single cell-derived colonies for the development of more stable and uniform monoclonal cell lines, for example for the production of recombinant proteins for human therapeutic use. The method is applicable for many cell lines, because HEK293 cells (used here) are more sensitive and "harder-to-clone" than many other mammalian cell lines including CHO and BHK. The method of the invention, as exemplified here, also offers the advantage that the image analyses can be done at any time, because all images and cell / colony coordinates are stored in a computer or suitable storage medium by the respective software. This can also facilitate the decision if a high producer cell line needs to be re-cloned.

### Claims

1.   A method for picking a colony of cells, the method comprising either the steps of

(a) providing a suspension comprising cells and semi-solid medium in a sample container, the sample container having a substantially flat bottom wall,
(b) centrifuging the sample container such that, after centrifugation, substantially all cells are positioned at the bottom wall,
(c) determining and recording the position of one or more cells located within at least one predetermined region at the bottom wall,
(d) incubating the sample container under conditions suitable for the growth of colonies of cells,
(e) determining and recording the area occupied by one or more colonies of cells, the one or more colonies of cells being located within the at least one predetermined region at the bottom wall, and
(f) selecting and picking at least one of the one or more colonies of cells occupying the area or areas determined and recorded in (e), and
(g) optionally, inferring that a colony of cells selected and picked in (f) is monoclonal if the position of not less and not more than one cell, as determined and recorded in (c), is located within the area determined and recorded in (e) as the area occupied by the colony of cells selected and picked in (f);

or the steps of

(a') providing a suspension comprising cells and semi-solid medium in a sample container, the sample container having a substantially flat bottom wall,

(b') centrifuging the sample container such that, after centrifugation, substantially all cells are positioned at the bottom wall,

(c') determining and recording the position of one or more cells located within at least one predetermined region at the bottom wall,

(d') incubating the sample container under conditions suitable for the growth of colonies of cells,

(e') determining and recording the area occupied by one or more colonies of cells, the one or more colonies of cells being located within the at least one predetermined region at the bottom wall,

(f') inferring that a colony of cells is monoclonal if the position of not less and not more than one cell, as determined and recorded in (c'), is located within the area determined and recorded in (e') as the area occupied by the colony of cells, and

(g') selecting and picking at least one monoclonal colony of cells.

2. A method for producing a cell line, the method comprising either the steps of

(a) providing a suspension comprising cells and semi-solid medium in a sample container, the sample container having a substantially flat bottom wall,

(b) centrifuging the sample container such that, after centrifugation, substantially all cells are positioned at the bottom wall,

(c) determining and recording the position of one or more cells located within at least one predetermined region at the bottom wall,

(d) incubating the sample container under conditions suitable for the growth of colonies of cells,

(e) determining and recording the area occupied by one or more colonies of cells, the one or more colonies of cells being located within the at least one predetermined region at the bottom wall,

(f) selecting and picking at least one of the one or more colonies of cells occupying the area or areas determined and recorded in (e), and

(g) producing a cell line by culturing under suitable conditions a colony of cells selected and picked in (f), and

(h) optionally, inferring that the cell line is monoclonal if the position of not less and not more than one cell, as determined and recorded in (c), is located within the area determined and recorded in (e) as the area occupied by the colony of cells selected and picked in (f) and cultured to establish the cell line in (g);

or the steps of

(a') providing a suspension comprising cells and semi-solid medium in a sample container, the sample container having a substantially flat bottom wall,

(b') centrifuging the sample container such that, after centrifugation, substantially all cells are positioned at the bottom wall,

(c') determining and recording the position of one or more cells located within at least one predetermined region at the bottom wall,

(d') incubating the sample container under conditions suitable for the growth of colonies of cells,

(e') determining and recording the area occupied by one or more colonies of cells, the one or more colonies of cells being located within the at least one predetermined region at the bottom wall,

(f') selecting and picking at least one of the one or more colonies of cells occupying the area or areas determined and recorded in (e'),

(g') inferring that a colony of cells selected and picked in (f') is monoclonal if the position of not less and not more than one cell, as determined and recorded in (c'), is located within the area determined and recorded in (e') as the area occupied by the colony of cells selected and picked in (f'), and

(h') producing a monoclonal cell line by culturing under suitable conditions a colony of cells selected and picked in (f') and inferred to be monoclonal in (g').

3. The method according to claim 2, wherein, in (g) or (h'), the colony of cells is cultured at 32 - 37 °C and 3 - 12% $CO_2$, preferably 32 - 37 °C and 5 - 10% $CO_2$, more preferably 37 °C and 5 - 7% $CO_2$, in humidified atmosphere in carbonate-buffered growth medium for approximately 3 days to 1 month.

4. The method according to any one of the preceding claims, wherein the cells are animal cells, human cells, or insect cells, preferably wherein the cells are mammalian cells, more preferably wherein the cells are HEK293, CHO, BHK, stem cells, continuous cell lines, primary cells, cancer cell lines, cell lines immortalized by any means, hybridomas of any species, NSO, Sp2/0, HKB11, HuH7, HepG2, SkHep, Vero, Cos, or PerC6, all of them growing adherently, or adapted to growing in suspension and/or growing in suspension.

5. The method according to any one of the preceding claims, wherein, in (c) or (c'), the position of the one or more cells is determined and recorded by recording a first image of the cells,

wherein a horizontal plane, parallel to the bottom wall and intersecting the cells above the bottom wall, is the focal plane of the first image,
such that the one or more cells located within at least one predetermined region at the bottom wall can be recognized in the first image,
and such that coordinates or any other unique identifier can be assigned to the one or more cells in the first image.

6. The method according to claim 5, wherein, in (e) or (e'), the area occupied by the one or more colonies of cells is determined and recorded by recording a second image of the cells,

wherein a horizontal plane, parallel to the bottom wall and intersecting the cells above the bottom wall, is the focal plane of the second image,
such that the one or more colonies of cells can be recognized in the second image,
and such that coordinates or any other unique identifier can be assigned to the one or more colonies of cells in the second image and these coordinates or unique identifier can be compared to any coordinates or unique identifier assigned to the one or more cells in the first image.

7. The method according to claim 6, wherein recording the second image and selecting and picking of a colony of cells is performed using a colony picking apparatus, preferably wherein the colony picking apparatus is capable of recording brightfield and fluorescent microscopic images, more preferably wherein recording the second image and selecting and picking of a colony of cells is performed using the ClonePix FL system or the ClonePix 2 system.

8. The method according to any one of the preceding claims, wherein the selecting and picking of a colony of cells is based on the presence and intensity of fluorescence of the colony itself and/or directly surrounding the colony, and/or on sufficient distance to the next colony.

9. The method according to any one of claims 5 to 8, wherein, during the incubation time in (d) or (d'), one or more additional image(s) is or are recorded of the cells,

wherein a horizontal plane, parallel to the bottom wall and intersecting the cells above the bottom wall, is the focal plane of the one or more additional image(s),
wherein cells and/or colonies of cells can be recognized in the additional image(s),
and wherein coordinates or any other unique identifier can be assigned to cells and/or colonies of cells in the additional image(s) such that these coordinates or unique identifier can be compared to any coordinates or unique identifier assigned to the one or more cells in the first image,
preferably wherein the additional images are recorded once a day.

10. The method according to any one of claims 5 to 9, wherein the image(s) is or are recorded using light microscopy and a camera.

11. The method according to any one of claims 6 to 10, wherein the first image and any additional image(s), but not the second image, is or are recorded using an imaging cytometer, preferably wherein the imaging cytometer is capable of recording brightfield microscopic images of the cell culture, more preferably wherein the imaging cytometer is a Celigo cytometer.

12. The method according to any one of the preceding claims, wherein, after (b) or (b') but before (c) or (c'), the method further comprises a step of choosing the at least one predetermined region at the bottom wall based on a distribution of cells in the sample container, preferably wherein the step of choosing the at least one predetermined region at the bottom wall is carried out with the help of a light microscope or with the help of a light microscope and a computer, more preferably wherein the light microscope is an imaging cytometer, such as a Celigo cytometer, or a colony picking apparatus, such as a ClonePix FL system or a ClonePix 2 system.

13. The method according to any one of the preceding claims,

wherein the at least one predetermined region at the bottom wall corresponds to the entire surface area of the bottom wall which is in contact with the suspension;

and/or wherein, in (c) or (c'), the position of each cell located within the at least one predetermined region at the bottom wall is determined and recorded;

and/or wherein, in (a) or (a'), a concentration of the cells in the suspension is 250 to 1000 cells/ml if the cells are stable serum-free cell lines, 50 to 500 cells/ml if the cells are stable serum-containing cell lines, 1000 to 5000 cells/ml if the cells are obtained by serum-free transfection, 500 to 2000 cells/ml if the cells are obtained by serum-containing transfection, $10^5$ to $10^6$ cells/ml if the cells are obtained by hybridoma fusion, or 1000 cells/ml if the cells are HEK293 cells;

and/or wherein at least one of the one or more colonies of cells occupying the area or areas determined and recorded in (e) or (e') is stained with a contrast enhancing agent in order to assist in selecting and/or picking the colony;

and/or wherein at least one of the one or more colonies of cells occupying the area or areas determined and recorded in (e) or (e') is stained with a fluorescent dye in order to assist in selecting and/or picking the colony.

14. The method according to any one of the preceding claims, wherein the cells of at least one of the one or more colonies of cells occupying the area or areas determined and recorded in (e) or (e') comprise, transcribe, translate, replicate, express, display on their surface, express intracellularly, and/or secrete a biological molecule of interest or a biological entity of interest;

preferably wherein the biological molecule of interest is selected from the group consisting of: a protein, a polypeptide, a peptide, a nucleic acid, and a part thereof, or wherein the biological entity of interest is selected from the group consisting of: a virus particle, a viral vector, and a part thereof.

15. The method according to any one of the preceding claims, wherein

(i) the semi-solid medium is based on methylcellulose,
(ii) the semi-solid medium contains one or more additives selected from the group consisting of: monoclonal or polyclonal antibodies, or fragments thereof, labelled with a fluorescent dye; proteins, such as receptors or ligands, labelled with a fluorescent dye; fluorescent dyes specific for a cellular component such as for nucleic acids; and fluorogenic enzyme substrates or enzyme inhibitors, such as fluorescein isothiocyanate (FITC)-labelled methotrexate (MTX),
(iii) the sample container is a 1-well plate or petri dish, 6-well plate, 12-well plate, 24-well plate, 48-well plate, 96-well plate, 384-well plate, or a 1536-well plate,
(iv) in (b) or (b'), the centrifuging is performed at 1000 x g for 10 minutes,
(v) after (b) or (b'), the cells are capable of dividing, and/or
(vi) in (d) or (d'), the sample container is incubated at 32 - 37 °C and 3 - 12% $CO_2$, preferably 32 - 37 °C and 5 - 10% $CO_2$, more preferably 37 °C and 5 - 7% $CO_2$, in humidified atmosphere for approximately 3 days to 1 month.

**Patentansprüche**

1. Verfahren zum Picken einer Zellkolonie, wobei das Verfahren entweder die folgenden Schritte umfasst:

(a) Bereitstellen einer Suspension, die Zellen und halbfestes Medium umfasst, in einem Probenbehälter, wobei der Probenbehälter einen Boden hat, der im Wesentlichen flach ist,
(b) Zentrifugieren des Probenbehälters, so dass nach der Zentrifugation im Wesentlichen alle Zellen auf dem Boden positioniert sind,
(c) Ermitteln und Aufnehmen der Position von einer oder mehreren Zellen, die innerhalb mindestens einer vorherbestimmten Region auf dem Boden lokalisiert sind,
(d) Inkubieren des Probenbehälters unter Bedingungen, die für das Wachstum von Zellkolonien geeignet sind,
(e) Ermitteln und Aufnehmen des Bereichs, der von einer oder mehreren Zellkolonien besetzt ist, wobei die eine oder mehreren Zellkolonien innerhalb der mindestens einen vorherbestimmten Region auf dem Boden lokalisiert sind, und
(f) Auswählen und Picken von mindestens einer der einen oder mehreren Zellkolonien, die den Bereich oder die Bereiche, die in (e) ermittelt und aufgenommen wurden, besetzen, und
(g) optional, Schlussfolgern, dass eine Zellkolonie, die in (f) ausgewählt und gepickt wurde monoklonal ist, wenn die Position von nicht weniger und nicht mehr als einer Zelle, wie ermittelt und aufgenommen in (c), innerhalb des Bereichs lokalisiert ist, der in (e) als der Bereich ermittelt und aufgenommen wurde, der von der Zellkolonie, die in (f) ausgewählt und gepickt wurde, besetzt ist;

oder die folgenden Schritte:

(a') Bereitstellen einer Suspension, die Zellen und halbfestes Medium umfasst, in einem Probenbehälter, wobei der Probenbehälter einen Boden hat, der im Wesentlichen flach ist,
(b') Zentrifugieren des Probenbehälters, so dass nach der Zentrifugation im Wesentlichen alle Zellen auf dem Boden positioniert sind,
(c') Ermitteln und Aufnehmen der Position von einer oder mehreren Zellen, die innerhalb mindestens einer vorherbestimmten Region auf dem Boden lokalisiert sind,
(d') Inkubieren des Probenbehälters unter Bedingungen, die für das Wachstum von Zellkolonien geeignet sind,
(e') Ermitteln und Aufnehmen des Bereichs, der von einer oder mehreren Zellkolonien besetzt ist, wobei die eine oder mehreren Zellkolonien innerhalb der mindestens einen vorherbestimmten Region auf dem Boden lokalisiert sind,
(f') Schlussfolgern, dass eine Zellkolonie monoklonal ist, wenn die Position von nicht weniger und nicht mehr als einer Zelle, wie ermittelt und aufgenommen in (c'), innerhalb des Bereichs lokalisiert ist, der in (e') als der Bereich ermittelt und aufgenommen wurde, der von der Zellkolonie besetzt ist, und
(g') Auswählen und Picken von mindestens einer monoklonalen Zellkolonie.

2. Verfahren zum Herstellen einer Zelllinie, wobei das Verfahren entweder die folgenden Schritte umfasst:

(a) Bereitstellen einer Suspension, die Zellen und halbfestes Medium umfasst, in einem Probenbehälter, wobei der Probenbehälter einen Boden hat, der im Wesentlichen flach ist,
(b) Zentrifugieren des Probenbehälters, so dass nach der Zentrifugation im Wesentlichen alle Zellen auf dem Boden positioniert sind,
(c) Ermitteln und Aufnehmen der Position von einer oder mehreren Zellen, die innerhalb mindestens einer vorherbestimmten Region auf dem Boden lokalisiert sind,
(d) Inkubieren des Probenbehälters unter Bedingungen, die für das Wachstum von Zellkolonien geeignet sind,
(e) Ermitteln und Aufnehmen des Bereichs, der von einer oder mehreren Zellkolonien besetzt ist, wobei die eine oder mehreren Zellkolonien innerhalb der mindestens einen vorherbestimmten Region auf dem Boden lokalisiert sind,
(f) Auswählen und Picken von mindestens einer der einen oder mehreren Zellkolonien, die den Bereich oder die Bereiche, die in (e) ermittelt und aufgenommen wurden, besetzen, und
(g) Herstellen einer Zelllinie durch das Kultivieren einer Zellkolonie, die in (f) ausgewählt und gepickt wurde, unter geeigneten Bedingungen, und
(h) Optional, Schlussfolgern, dass die Zelllinie monoklonal ist, wenn die Position von nicht weniger und nicht mehr als einer Zelle, wie ermittelt und aufgenommen in (c), innerhalb des Bereichs lokalisiert ist, der in (e) als der Bereich ermittelt und aufgenommen wurde, der von der Zellkolonie besetzt ist, die in (f) ausgewählt und gepickt und in (g) kultiviert wurde, um eine Zelllinie zu etablieren;

oder die folgenden Schritte umfasst:

(a') Bereitstellen einer Suspension, die Zellen und halbfestes Medium umfasst, in einem Probenbehälter, wobei der Probenbehälter einen Boden hat, der im Wesentlichen flach ist,
(b') Zentrifugieren des Probenbehälters, so dass nach der Zentrifugation im Wesentlichen alle Zellen auf dem Boden positioniert sind,
(c') Ermitteln und Aufnehmen der Position von einer oder mehreren Zellen, die innerhalb mindestens einer vorherbestimmten Region auf dem Boden lokalisiert sind,
(d') Inkubieren des Probenbehälters unter Bedingungen, die für das Wachstum von Zellkolonien geeignet sind,
(e') Ermitteln und Aufnehmen des Bereichs, der von einer oder mehreren Zellkolonien besetzt ist, wobei die eine oder mehreren Zellkolonien innerhalb der mindestens einen vorherbestimmten Region auf dem Boden lokalisiert sind,
(f') Auswählen und Picken von mindestens einer der einen oder mehreren Zellkolonien, die den Bereich oder die Bereiche, die in (e') ermittelt und aufgenommen wurden, besetzen,
(g') Schlussfolgern, dass eine Zellkolonie, die in (f') ausgewählt und gepickt wurde, monoklonal ist, wenn die Position von nicht weniger und nicht mehr als einer Zelle, wie ermittelt und aufgenommen in (c'), innerhalb des Bereichs lokalisiert ist, der in (e') als der Bereich ermittelt und aufgenommen wurde, der von der Zellkolonie besetzt ist, die in (f') ausgewählt und gepickt wurde, und

(h') Herstellen einer monoklonalen Zelllinie durch Kultivieren einer Zellkolonie, die in (f') ausgewählt und gepickt wurde und über welche in (g') geschlussfolgert wurde, dass sie monoklonal ist, unter geeigneten Bedingungen.

3. Verfahren gemäß Anspruch 2, wobei in (g) oder (h') die Zellkolonie bei 32 - 37 °C und 3 - 12% $CO_2$, bevorzugt 32 - 37 °C und 5 - 10% $CO_2$, bevorzugter 37 °C und 5 - 7% $CO_2$, in einer befeuchteten Atmosphäre in Carbonatgepuffertem Wachstumsmedium für ungefähr 3 Tage bis 1 Monat kultiviert wird.

4. Verfahren gemäß irgendeinem der vorherstehenden Ansprüche, wobei die Zellen Tierzellen, Menschenzellen oder Insektenzellen sind, bevorzugt wobei die Zellen Säugetierzellen sind, bevorzugter wobei die Zellen HEK293, CHO, BHK, Stammzellen, kontinuierliche Zelllinien, primäre Zellen, Krebszelllinien, Zelllinien, die durch jegliche Mittel immortalisiert wurden, Hybridomas von irgendeiner Spezies, NSO, Sp2/0, HKB11, HuH7, HepG2, SkHep, Vero, Cos oder PerC6 sind, wobei alle von diesen anhaftend wachsen oder angepasst wurden um in Suspensionen zu wachsen und/oder in Suspensionen wachsen.

5. Verfahren gemäß irgendeinem der vorherstehenden Ansprüche, wobei in (c) oder (c') die Position der einen oder mehreren Zellen durch das Aufnehmen eines ersten Bildes der Zellen ermittelt und aufgenommen wird,

wobei eine horizontale Ebene, die parallel zum Boden und die Zellen durchkreuzend über dem Boden liegt, die Fokalebene des ersten Bilds ist,
so dass die eine oder mehreren Zellen, die in der vorherbestimmten Region auf dem Boden lokalisiert sind, in dem ersten Bild erkannt werden können,
und so dass Koordinaten oder irgendein anderer einzigartiger Identifikator der einen oder den mehreren Zellen in dem ersten Bild zugeordnet werden können.

6. Verfahren gemäß Anspruch 5, wobei in (e) oder (e') der Bereich, der von der einen oder den mehreren Zellkolonien besetzt wird, durch die Aufnahme eines zweiten Bildes der Zellen ermittelt und aufgenommen wird,

wobei eine horizontale Ebene, die parallel zum Boden und die Zellen durchkreuzend über dem Boden liegt, die Fokalebene des zweiten Bilds ist,
so dass die eine oder mehreren Zellkolonien in dem zweiten Bild erkannt werden können,
und so dass Koordinaten oder irgendein anderer einzigartiger Identifikator der einen oder den mehreren Zellkolonien in dem zweiten Bild zugeordnet werden können, und diese Koordinaten oder anderer einzigartiger Identifikator mit irgendwelchen Koordinaten oder einzigartigem Identifikator verglichen werden können, die der einen oder den mehreren Zellen in dem ersten Bild zugeordnet wurden.

7. Verfahren gemäß Anspruch 6, wobei das Aufnehmen des zweiten Bildes und das Auswählen und Picken einer Zellkolonie unter Verwendung von einer Kolonie-pickenden Vorrichtung ausgeführt wird, bevorzugt wobei die Kolonie-pickende Vorrichtung die Fähigkeit besitzt, Hellfeld- und fluoreszente mikroskopische Bilder aufzunehmen, bevorzugter wobei das Aufnehmen des zweiten Bilds und das Auswählen und Picken einer Zellkolonie unter Verwendung des ClonePix FL Systems oder des ClonePix 2 Systems ausgeführt wird.

8. Verfahren gemäß irgendeinem der vorherstehenden Ansprüche, wobei das Auswählen und Picken einer Zellkolonie auf dem Auftreten und der Intensität von Fluoreszenz der Kolonie selbst und/oder in der direkten Umgebung der Kolonie und/oder auf ausreichender Distanz zu der nächsten Kolonie basiert ist.

9. Verfahren gemäß irgendeinem der Ansprüche 5 bis 8, wobei während der Inkubationszeit in (d) oder (d') ein oder mehrere zusätzliche Bild(er) der Zellen aufgenommen wird oder werden,

wobei eine horizontale Ebene, die parallel zum Boden und die Zellen durchkreuzend über dem Boden liegt, die Fokalebene des einen oder der mehreren zusätzlichen Bilds(er) ist,
wobei Zellen und/oder Zellkolonien in dem (den) zusätzlichen Bild(ern) erkannt werden können,
und wobei Koordinaten oder irgendein anderer einzigartiger Identifikator den Zellen und/oder Zellkolonien in dem (den) zusätzlichen Bild(ern) zugeordnet werden können, so dass diese Koordinaten oder einzigartiger Identifikator mit irgendwelchen Koordinaten oder einzigartigem Identifikator verglichen werden können, die der einen oder den mehreren Zellen in dem ersten Bild zugeordnet wurden,
bevorzugt wobei die zusätzlichen Bilder einmal pro Tag aufgenommen werden.

10. Verfahren gemäß irgendeinem der Ansprüche 5 bis 9, wobei das (die) Bild(er) mittels Lichtmikroskopie und einer Kamera aufgenommen wird oder werden.

11. Verfahren gemäß irgendeinem der Ansprüche 6 bis 10, wobei das erste Bild und jedes (der) zusätzliche(n) Bild(er), aber nicht das zweite Bild, mittels eines bildgebenden Zytometers aufgenommen wird oder werden, bevorzugt wobei das bildgebende Zytometer die Fähigkeit besitzt, Hellfeld mikroskopische Bilder der Zellkultur aufzunehmen, bevorzugter wobei das bildgebende Zytometer ein Celigo Zytometer ist.

12. Verfahren gemäß irgendeinem der vorherstehenden Ansprüche, wobei nach (b) oder (b') aber vor (c) oder (c') das Verfahren weiter einen Schritt des Aussuchens der mindestens einen vorherbestimmten Region auf dem Boden umfasst, basierend auf einer Verteilung von Zellen in dem Probenbehälter, bevorzugt wobei der Schritt des Aussuchens der mindestens einen vorherbestimmten Region auf dem Boden mit Hilfe eines Lichtmikroskops oder mit Hilfe eines Lichtmikroskops und eines Computers ausgeführt wird, bevorzugter wobei das Lichtmikroskop ein bildgebendes Zytometer ist, wie beispielsweise ein Celigo Zytometer oder eine Kolonie-pickende Vorrichtung ist, wie beispielsweise ein ClonePix FL System oder ein ClonePix 2 System.

13. Verfahren gemäß irgendeinem der vorherstehenden Ansprüche,

   wobei die mindestens eine vorherbestimmte Region auf dem Boden der gesamten Oberfläche des Bodens, die mit der Suspension in Kontakt ist, entspricht;
   und/oder wobei in (c) oder (c') die Position jeder Zelle, die innerhalb der mindestens einen vorherbestimmten Region auf dem Boden lokalisiert ist, ermittelt und aufgenommen wird;
   und/oder wobei in (a) oder (a') eine Konzentration der Zellen in der Suspension 250 bis 1000 Zellen/ml ist falls die Zellen stabile Serum-freie Zelllinien sind, 50 bis 500 Zellen/ml falls die Zellen stabile Serum-enthaltende Zelllinien sind, 1000 bis 5000 Zellen/ml falls die Zellen durch Serum-freie Transfektion erhalten werden, 500 bis 2000 Zellen/ml falls die Zellen durch Serum-enthaltende Transfektion erhalten werden, $10^5$ bis $10^6$ Zellen/ml falls die Zellen durch Hybridoma-Fusion erhalten werden, oder 1000 Zellen/ml falls die Zellen HEK293 Zellen sind;
   und/oder wobei mindestens eine der einen oder mehreren Zellkolonien, die den Bereich oder die Bereiche besetzen, die in (e) oder (e') ermittelt und aufgenommen wurden, mit einem kontrasterhöhenden Mittel gefärbt wird, um die Auswahl und/oder das Picken der Kolonie zu unterstützen;
   und/oder wobei mindestens eine der einen oder mehreren Zellkolonien, die den Bereich oder die Bereiche besetzen, die in (e) oder (e') ermittelt und aufgenommen wurden, mit einer fluoreszenten Farbe gefärbt wird, um die Auswahl und/oder das Picken der Kolonie zu unterstützen.

14. Verfahren gemäß irgendeinem der vorherstehenden Ansprüche, wobei die Zellen von mindestens einer der einen oder mehreren Zellkolonien, die den Bereich oder die Bereiche besetzen, die in (e) oder (e') ermittelt und aufgenommen wurden ein biologisches Molekül von Interesse oder eine biologische Einheit von Interesse umfassen, transkribieren, translatieren, replizieren, exprimieren, auf ihrer Oberfläche tragen, intrazellulär exprimieren, und/oder absondern;
   bevorzugt wobei das biologische Molekül von Interesse ausgewählt wird aus der Gruppe bestehend aus: einem Protein, einem Polypeptid, einem Peptid, einer Nukleinsäure, und einem Teil davon oder wobei die biologische Einheit von Interesse ausgewählt wird aus der Gruppe bestehend aus: einem Viruspartikel, einem viralen Vektor, und einem Teil davon.

15. Verfahren gemäß irgendeinem der vorherstehenden Ansprüche, wobei

   (i) das halbfeste Medium auf Methylzellulose basiert ist,
   (ii) das halbfeste Medium einen oder mehrere Zusatzstoffe enthält, ausgewählt aus der Gruppe bestehend aus: monoklonalen oder polyklonalen Antikörpern oder Fragmenten davon, die mit einer fluoreszenten Farbe markiert sind; Proteine, wie beispielsweise Rezeptoren oder Liganden, die mit einer fluoreszenten Farbe markiert sind; fluoreszenten Farben, die für eine zelluläre Komponente spezifisch sind, wie beispielsweise für Nukleinsäuren; und fluorogene Enzym-Substrate oder Enzym-Hemmstoffe, wie beispielsweise Fluoreszein-Isothiocyanat (FITC)-markiertes Methotrexat (MTX),
   (iii) der Probenbehälter ein 1-Well Platte oder eine Petrischale, 6-Well Platte, 12-Well Platte, 24-Well Platte, 48-Well Platte, 96-Well Platte, 384-Well Platte oder eine 1536-Well Platte ist,
   (iv) das Zentrifugieren in (b) oder (b') bei 1000 x g für 10 min ausgeführt wird,
   (v) die Zellen nach (b) oder (b') die Fähigkeit haben, sich zu teilen, und/oder
   (vi) Der Probenbehälter in (d) oder (d') bei 32 - 37 °C und 3 - 12% $CO_2$, bevorzugt 32 - 37 °C und 5 - 10% $CO_2$,

bevorzugter 37 °C und 5 - 7% $CO_2$, in einer befeuchteten Atmosphäre für ungefähr 3 Tage bis 1 Monat inkubiert wird.

**Revendications**

1. Procédé de repiquage d'une colonie de cellules, le procédé comprenant l'une ou l'autre des étapes suivantes

(a) la fourniture d'une suspension comprenant des cellules et un milieu semi-solide dans un récipient pour échantillon, le récipient pour échantillon présentant une paroi de fond sensiblement plate,
(b) la centrifugation du récipient pour échantillon de telle sorte que, après centrifugation, sensiblement toutes les cellules soient positionnées au niveau de la paroi de fond,
(c) la détermination et l'enregistrement de la position d'une ou de plusieurs cellules situées dans au moins une région prédéterminée au niveau de la paroi de fond,
(d) la mise en incubation du récipient pour échantillon dans des conditions appropriées pour la croissance de colonies de cellules,
(e) la détermination et l'enregistrement de la zone occupée par une ou plusieurs colonies de cellules, les une ou plusieurs colonies de cellules étant situées dans la au moins une région prédéterminée au niveau de la paroi de fond, et
(f) la sélection et le repiquage d'au moins l'une des une ou plusieurs colonies de cellules occupant la zone ou les zones déterminées et enregistrées dans (e), et
(g) facultativement, la déduction qu'une colonie de cellules sélectionnée et repiquée dans (f) est monoclonale si la position de pas moins et de pas plus d'une cellule, telle que déterminée et enregistrée dans (c), est située dans la zone déterminée et enregistrée dans (e) comme étant la zone occupée par la colonie de cellules sélectionnées et repiquée dans (f) ;

ou les étapes suivantes

(a') la fourniture d'une suspension comprenant des cellules et un milieu semi-solide dans un récipient pour échantillon, le récipient pour échantillon présentant une paroi de fond sensiblement plate,
(b') la centrifugation du récipient pour échantillon de telle sorte que, après centrifugation, sensiblement toutes les cellules soient positionnées au niveau de la paroi de fond,
(c') la détermination et l'enregistrement de la position d'une ou de plusieurs cellules situées dans au moins une région prédéterminée au niveau de la paroi de fond, (d') la mise en incubation du récipient pour échantillon dans des conditions appropriées pour la croissance de colonies de cellules,
(e') la détermination et l'enregistrement de la zone occupée par une ou plusieurs colonies de cellules, les une ou plusieurs colonies de cellules étant situées dans la au moins une région prédéterminée au niveau de la paroi de fond,
(f') la déduction qu'une colonie de cellules est monoclonale si la position de pas moins et de pas plus d'une cellule, telle que déterminée et enregistrée dans (c'), est située dans la zone déterminée et enregistrée dans (e') comme étant la zone occupée par la colonie de cellules, et
(g') la sélection et le repiquage d'au moins une colonie monoclonale de cellules.

2. Procédé de production d'une lignée cellulaire, le procédé comprenant l'une ou l'autre des étapes suivantes

(a) la fourniture d'une suspension comprenant des cellules et un milieu semi-solide dans un récipient pour échantillon, le récipient pour échantillon présentant une paroi de fond sensiblement plate,
(b) la centrifugation du récipient pour échantillon de telle sorte que, après centrifugation, sensiblement toutes les cellules soient positionnées au niveau de la paroi de fond,
(c) la détermination et l'enregistrement de la position d'une ou de plusieurs cellules situées dans au moins une région prédéterminée au niveau de la paroi de fond,
(d) la mise en incubation du récipient pour échantillon dans des conditions appropriées pour la croissance de colonies de cellules,
(e) la détermination et l'enregistrement de la zone occupée par une ou plusieurs colonies de cellules, les une ou plusieurs colonies de cellules étant situées dans la au moins une région prédéterminée au niveau de la paroi de fond,
(f) la sélection et le repiquage d'au moins l'une des une ou plusieurs colonies de cellules occupant la zone ou les zones déterminées et enregistrées dans (e), et

(g) la production d'une lignée cellulaire par la mise en culture dans des conditions appropriées d'une colonie de cellules sélectionnée et repiquée dans (f), et

(h) facultativement, la déduction que la lignée cellulaire est monoclonale si la position de pas moins et de pas plus d'une cellule, telle que déterminée et enregistrée dans (c), est située dans la zone déterminée et enregistrée dans (e) comme étant la zone occupée par la colonie de cellules sélectionnées et repiquée dans (f) et mise en culture pour établir la lignée cellulaire dans (g) ;

ou les étapes suivantes

(a') la fourniture d'une suspension comprenant des cellules et un milieu semi-solide dans un récipient pour échantillon, le récipient pour échantillon présentant une paroi de fond sensiblement plate,

(b') la centrifugation du récipient pour échantillon de telle sorte que, après centrifugation, sensiblement toutes les cellules soient positionnées au niveau de la paroi de fond,

(c') la détermination et l'enregistrement de la position d'une ou de plusieurs cellules situées dans au moins une région prédéterminée au niveau de la paroi de fond,

(d') la mise en incubation du récipient pour échantillon dans des conditions appropriées pour la croissance de colonies de cellules,

(e') la détermination et l'enregistrement de la zone occupée par une ou plusieurs colonies de cellules, les une ou plusieurs colonies de cellules étant situées dans la au moins une région prédéterminée au niveau de la paroi de fond,

(f') la sélection et le repiquage d'au moins l'une des une ou plusieurs colonies de cellules occupant la zone ou les zones déterminées et enregistrées dans (e'),

(g') la déduction qu'une colonie de cellules sélectionnées et repiquée dans (f') est monoclonale si la position de pas moins et de pas plus d'une cellule, telle que déterminée et enregistrée dans (c'), est située dans la zone déterminée et enregistrée dans (e') comme étant la zone occupée par la colonie de cellules sélectionnées et repiquée dans (f'), et

(h') la production d'une lignée cellulaire monoclonale par la mise en culture dans des conditions appropriées d'une colonie de cellules sélectionnée et repiquée dans (f') et supposée être monoclonale dans (g').

3. Procédé selon la revendication 2, dans lequel, dans (g) ou (h'), la colonie de cellules est mise en culture à 32 à 37 °C et 3 à 12 % de $CO_2$, de préférence à 32 à 37 °C et 5 à 10 % de $CO_2$, plus préférentiellement à 37 °C et 5 à 7 % de $CO_2$, dans une atmosphère humidifiée dans un milieu de croissance tamponné au carbonate pendant approximativement 3 jours à 1 mois.

4. Procédé selon l'une quelconque des revendications précédentes, dans lequel les cellules sont des cellules animales, des cellules humaines, ou des cellules d'insecte, de préférence dans lequel les cellules sont des cellules de mammifère, plus préférentiellement dans lequel les cellules sont HEK293, CHO, BHK, des cellules souches, des lignées cellulaires continues, des cellules primaires, des lignées cellulaires cancéreuses, des lignées cellulaires immortalisées par n'importe quel moyen, des hybridomes de n'importe quelle espèce, NSO, Sp2/0, HKB11, HuH7, HepG2, SkHep, Vero, Cos, ou PerC6, toutes ayant une croissance de manière adhérente, ou étant adaptées à une croissance en suspension et/ou croissant en suspension.

5. Procédé selon l'une quelconque des revendications précédentes, dans lequel, dans (c) ou (c'), la position des une ou plusieurs cellules est déterminée et enregistrée en enregistrant une première image des cellules, dans lequel un plan horizontal, parallèle à la paroi de fond et croisant les cellules au-dessus de la paroi de fond, est le plan focal de la première image, de sorte que les une ou plusieurs cellules situées dans au moins une région prédéterminée au niveau de la paroi de fond peuvent être reconnues sur la première image, et de sorte que des coordonnées ou tout autre identifiant unique peuvent être attribués aux une ou plusieurs cellules sur la première image.

6. Procédé selon la revendication 5, dans lequel, dans (e) ou (e'), la zone occupée par une ou plusieurs colonies de cellules est déterminée et enregistrée en enregistrant une seconde image des cellules, dans lequel un plan horizontal, parallèle à la paroi de fond et croisant les cellules au-dessus de la paroi de fond, est le plan focal de la seconde image, de sorte que les une ou plusieurs colonies de cellules peuvent être reconnues sur la seconde image, et de sorte que des coordonnées ou tout autre identifiant unique peuvent être attribués aux une ou plusieurs colonies de cellules sur la seconde image et ces coordonnées ou cet identifiant unique peuvent être comparés à n'importe quelles coordonnées ou à n'importe quel identifiant unique attribués aux une ou plusieurs cellules sur la première image.

7. Procédé selon la revendication 6, dans lequel l'enregistrement de la seconde image et la sélection et le repiquage d'une colonie de cellules sont effectués en utilisant un appareil de repiquage de colonie, de préférence dans lequel l'appareil de repiquage de colonie est capable d'enregistrer des images microscopiques sur fond clair et fluorescentes, plus préférentiellement dans lequel l'enregistrement de la seconde image et la sélection et le repiquage d'une colonie de cellules sont effectuées en utilisant le système ClonePix FL ou le système ClonePix 2.

8. Procédé selon l'une quelconque des revendications précédentes, dans lequel la sélection et le repiquage d'une colonie de cellules sont basés sur la présence et l'intensité d'une fluorescence de la colonie elle-même et/ou entourant directement la colonie, et/ou sur une distance suffisante jusqu'à la prochaine colonie.

9. Procédé selon l'une quelconque des revendications 5 à 8, dans lequel, pendant la période d'incubation dans (d) ou (d'), une ou plusieurs image(s) supplémentaire(s) est ou sont enregistrée(s) des cellules,
dans lequel un plan horizontal, parallèle à la paroi de fond et croisant les cellules au-dessus de la paroi de fond, est le plan focal des une ou plusieurs image(s) supplémentaire(s),
dans lequel des cellules et/ou des colonies de cellules peuvent être reconnues sur la ou les image(s) supplémentaire(s),
et dans lequel des coordonnées ou tout autre identifiant unique peuvent être attribués à des cellules et/ou à des colonies de cellules sur la ou les image(s) supplémentaire(s) de sorte que ces coordonnées ou cet identifiant unique peuvent être comparés à n'importe quelles coordonnées ou à n'importe identifiant unique attribués aux une ou plusieurs cellules sur la première image,
de préférence dans lequel les images supplémentaires sont enregistrées une fois par jour.

10. Procédé selon l'une quelconque des revendications 5 à 9, dans lequel la ou les image(s) est ou sont enregistrée(s) en utilisant une microscopie optique et une caméra.

11. Procédé selon l'une quelconque des revendications 6 à 10, dans lequel la première image et toute image supplémentaire, mais pas la seconde image, est ou sont enregistrée(s) en utilisant un cytomètre d'imagerie, de préférence dans lequel le cytomètre d'imagerie est capable d'enregistrer des images microscopiques sur fond clair de la culture cellulaire, plus préférentiellement dans lequel le cytomètre d'imagerie est un cytomètre Celigo.

12. Procédé selon l'une quelconque des revendications précédentes, dans lequel, après (b) ou (b'), mais avant (c) ou (c'), le procédé comprend en outre une étape de choix de la au moins une région prédéterminée au niveau de la paroi de fond sur la base d'une distribution de cellules dans le récipient pour échantillon, de préférence dans lequel l'étape de choix de la au moins une région prédéterminée au niveau de la paroi de fond est réalisée à l'aide d'un microscope optique ou à l'aide d'un microscope optique et d'un ordinateur, plus préférentiellement dans lequel le microscope optique est un cytomètre d'imagerie, tel qu'un cytomètre Celigo, ou un appareil de repiquage de colonie, tel qu'un système ClonePix FL ou un système ClonePix 2.

13. Procédé selon l'une quelconque des revendications précédentes,
dans lequel la au moins une région prédéterminée au niveau de la paroi de fond correspond à toute la surface de la paroi de fond qui est en contact avec la suspension ;
et/ou dans lequel, dans (c) ou (c'), la position de chaque cellule située dans la au moins une région prédéterminée au niveau de la paroi de fond est déterminée et enregistrée ;
et/ou dans lequel, dans (a) ou (a'), une concentration des cellules dans la suspension est de 250 à 1000 cellules/ml si les cellules sont des lignées cellulaires stables sans sérum, de 50 à 500 cellules/ml si les cellules sont des lignées cellulaires stables contenant du sérum, 1000 à 5000 cellules/ml si les cellules sont obtenues par transfection sans sérum, 500 à 2000 cellules/ml si les cellules sont obtenues par transfection contenant du sérum, $10^5$ à $10^6$ cellules/ml si les cellules sont obtenues par fusion d'hybridomes, ou 1000 cellules/ml si les cellules sont des cellules HEK293 ;
et/ou dans lequel au moins une des une ou plusieurs colonies de cellules occupant la zone ou les zones déterminées et enregistrées dans (e) ou (e') est colorée avec un agent de renforcement de contraste afin de faciliter la sélection et/ou le repiquage de la colonie ;
et/ou dans lequel au moins une des une ou plusieurs colonies de cellules occupant la zone ou les zones déterminées et enregistrées dans (e) ou (e') est colorée avec un colorant fluorescent afin de faciliter la sélection et/ou le repiquage de la colonie.

**14.** Procédé selon l'une quelconque des revendications précédentes, dans lequel les cellules d'au moins une des une ou plusieurs colonies de cellules occupant la zone ou les zones déterminées et enregistrées dans (e) ou (e') comprennent, transcrivent, traduisent, répliquent, expriment, affichent sur leur surface, expriment au niveau intracellulaire, et/ou sécrètent une molécule biologique d'intérêt ou une entité biologique d'intérêt ;
de préférence dans lequel la molécule biologique d'intérêt est sélectionnée dans le groupe consistant en : une protéine, un polypeptide, un peptide, un acide nucléique et une partie de ceux-ci, ou dans lequel l'entité biologique d'intérêt est sélectionnée dans le groupe consistant en : une particule virale, un vecteur viral, et une partie de celle-ci.

**15.** Procédé selon l'une quelconque des revendications précédentes, dans lequel

(i) le milieu semi-solide est à base de méthylcellulose,
(ii) le milieu semi-solide contient un ou plusieurs additifs sélectionnés dans le groupe consistant en : des anticorps monoclonaux ou polyclonaux, ou des fragments de ceux-ci, marqués avec un colorant fluorescent ; des protéines, telles que des récepteurs ou des ligands, marquées avec un colorant fluorescent; des colorants fluorescents spécifiques d'un composant cellulaire tel que des acides nucléiques ; et des substrats fluorogènes d'enzyme ou des inhibiteurs d'enzyme, tels que le méthotrexate (MTX) marqué à l'isothiocyanate de fluorescéine (FITC),
(iii) le récipient pour échantillon est une plaque à 1 puits ou une boîte de Pétri, une plaque à 6 puits, une plaque à 12 puits, une plaque à 24 puits, une plaque à 48 puits, une plaque à 96 puits, une plaque à 384 puits, ou une plaque à 1536 puits,
(iv) dans (b) ou (b'), la centrifugation est effectuée à 1000 x g pendant 10 minutes,
(v) après (b) ou (b'), les cellules sont capables de se diviser, et/ou
(vi) dans (d) ou (d'), le récipient pour échantillon est mis en incubation à 32 à 37 °C et 3 à 12 % de $CO_2$, de préférence à 32 à 37 °C et 5 à 10 % de $CO_2$, plus préférentiellement à 37 °C et 5 à 7 % de $CO_2$, dans une atmosphère humidifiée pendant approximativement 3 jours à 1 mois.

Fig. 1

Fig. 2

**Fig. 3**

**Fig. 4**

**Fig. 5**

**Fig. 6**

Fig. 7

**Coordinates Clonepix**

**Coordinates Celigo**

**Fig. 8**

Fig. 9

Day 0

Day 2

Day 7

Day 14

Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LAI et al.** *Pharmaceuticals,* 2013, vol. 6, 579-603
- **YOSHIMOTO et al.** *Scientific Reports,* 2013, vol. 3, 1191
- **HOU et al.** *New Biotechnology,* 2014, vol. 31 (3), 214-220
- **DHARSHANAN S ; HUNG CS.** Molecular Devices ClonePix FL Application Guide for New Users. *Methods Mol Biol.,* 2014, vol. 1131, 105-12
- **DAVID M. PRESCOTT.** METHODS IN CELL BIOLOGY. Academic Press, 13 January 1977, vol. 14, 240f, , 258f